(19) **Europäisches Patentamt / European Patent Office / Office européen des brevets**

(11) **EP 4 643 659 A1**

(12) # EUROPEAN PATENT APPLICATION
published in accordance with Art. 153(4) EPC

(43) Date of publication:
**05.11.2025 Bulletin 2025/45**

(21) Application number: **23910683.4**

(22) Date of filing: **27.12.2023**

(51) International Patent Classification (IPC):
**A23P 10/30** (2016.01)    **A23P 10/35** (2016.01)
**A23L 33/135** (2016.01)

(52) Cooperative Patent Classification (CPC):
**A23L 33/135; A23P 10/30; A23P 10/35**

(86) International application number:
**PCT/CN2023/142218**

(87) International publication number:
**WO 2024/140764 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 30.12.2022  CN 202211741593
                30.12.2022  CN 202211734574
                30.12.2022  CN 202211734622

(71) Applicant: **Inner Mongolia Yili Industrial Group
Co. Ltd.**
**Hohhot, Inner Mongolia 010000 (CN)**

(72) Inventors:
• **HE, Jian**
  **Hohhot, Inner Mongolia 010000 (CN)**
• **HOU, Zhanqun**
  **Hohhot, Inner Mongolia 010000 (CN)**
• **GONG, Guangyi**
  **Hohhot, Inner Mongolia 010000 (CN)**
• **WANG, Caiyun**
  **Hohhot, Inner Mongolia 010000 (CN)**

(74) Representative: **HGF**
**HGF Limited**
**1 City Walk**
**Leeds LS11 9DX (GB)**

(54) **HEAT-RESISTANT AND SHEAR-RESISTANT MICROCAPSULE, AND PREPARATION METHOD THEREFOR AND USE THEREOF**

(57)  A heat-resistant and shear-resistant microcapsule, comprising a core material, a protein wrapping layer wrapping the core material, and a wall material wrapping the protein wrapping layer. The core material comprises grease. The wall material is formed by heating and curing a colloid. According to the microcapsule, first, an internal environment in which moisture and oxygen are isolated is provided by wrapping internal phase grease with a protein, and the survival rate and stability of active substances in a heat treatment process are improved by means of heat absorption in phase change at melting points; then, the wall material is used for further protection, so that the microcapsule has relatively high stability, has relatively strong shear resistance while having high temperature resistance, and also has relatively high acid resistance.

**EP 4 643 659 A1**

**Description**

**CROSS REFERENCE TO RELATED APPLICATIONS**

This application claims the priority of Chinese Patent Application No. 202211741593.8, filed with the China National Intellectual Property Administration on December 30, 2022, and titled with "HEAT-RESISTANT AND SHEAR-RESISTANT MICROCAPSULE, AND PREPARATION METHOD THEREFOR AND USE THEREOF", which is hereby incorporated by reference in its entirety.

This application claims the priority of Chinese Patent Application No. 202211734574.2, filed with the China National Intellectual Property Administration on December 30, 2022, and titled with "HIGH TEMPERATURE SHEAR RESISTANT COLLOID, MICROCAPSULE, PREPARATION METHOD AND USE THEREOF", which is hereby incorporated by reference in its entirety.

This application claims the priority of Chinese Patent Application No. 202211734622.8, filed with the China National Intellectual Property Administration on December 30, 2022, and titled with "HEAT-RESISTANT AND ACID-RESISTANT OIL AND FAT COMPOSITION, MICROCAPSULE, PREPARATION METHOD AND USE THEREOF", which is hereby incorporated by reference in its entirety.

**FIELD**

The present disclosure belongs to the field of food technology, and in particular relates to a heat-resistant and shear-resistant microcapsule, a production method and use thereof.

**BACKGROUND**

Microcapsules are micro-containers made of natural or synthetic polymers (wall materials). They can not only encapsulate and protect droplets, particles or gases (core materials) in the capsule, but also improve the performance and function of the core materials by selecting or producing suitable wall materials. Therefore, the microencapsulation method has great adaptability and advanced functions. Since microcapsules were invented in the 1930s, they have been intensively studied and significant progress has been made. They are now widely used in medicine, food, cosmetics, pesticides and fertilizers, dyes and pigments, coatings, adhesives, textiles, inks, additives and other fields.

After volatile, oxidizable, photosensitive and heat-sensitive substances are microencapsulated, they can avoid direct contact with light, heat or air, which can inhibit their volatilization and oxidation, and reduce photo-(heat-)sensitivity.

Vitamin A is an important drug for preventing and treating night blindness, and vitamin D2 promotes the intestinal absorption of calcium and phosphorus. However, they are easily decomposed under ultraviolet radiation and easily oxidized in the air, which reduces their effectiveness. The main function of vitamin C is to treat scurvy, but it is unstable and easily destroyed when heated. Therefore, microencapsulation of vitamins can increase the storage period of vitamin drugs.

Probiotics have many functions such as improving intestinal microecological balance, improving immunity, anti-tumor, and lowering cholesterol. However, in the processing and storage of commercial products, parameters such as processing mechanical force, temperature, water activity, oxygen content, and pH changes may affect the survival of probiotics. In addition, probiotics must remain active after being digested by the gastrointestinal tract (it is generally believed that the bacterial activity should be maintained at $10^6$ CFU/g or $10^6$ CFU/mL or above) in order to colonize in the intestine. In order for probiotics to survive during production, processing, storage, and gastrointestinal digestion in the body and exert their health benefits to the human body, it is necessary to design a certain encapsulation system such as microcapsules to help probiotics resist the external processing environment, and then transport them to the designated location in the digestive tract and successfully colonize them.

However, as food or pharmaceutical raw materials, they need to be sterilized at high temperatures during the preparation process. For example, if the temperature of the sterilization process is high to kill most microorganisms, the wall material will often melt, causing the active substances to separate into the outer layer and become inactivated by contact with high temperatures.

In addition, high-temperature stirring is often required during food processing. For example, in the production of processed cheese, the raw materials of the processed cheese need to be heated and stirred in a melting pot. Bioactive substances including probiotics and lactoferrin are easily inactivated due to high temperatures. Moreover, due to the existence of high-temperature stirring, ordinary microcapsule products embedded with bioactive substances will also be detached from the core material due to the shear force generated by the stirring action, causing the bioactive substances to become inactivated by contact with high temperatures. Therefore, how to ensure that bioactive substances can better withstand the harsh conditions of the food processing process and maintain their original biological activity to the maximum extent has become a technical problem that needs to be solved in the art.

## SUMMARY

In view of this, the technical problem to be solved by the present disclosure is to provide a heat-resistant and shear-resistant microcapsule, a production method and use thereof.

The present disclosure provides a heat-resistant and shear-resistant microcapsule, comprising a core material, a protein coating layer wrapping the core material, and a wall material wrapping the protein coating layer; the core material comprises oil and fat; and the wall material is formed by heat-curing of a colloid.

Preferably, the microcapsules comprise at least one of the following features:

(1) the oil and fat have a differential scanning calorimetry curve with an endothermic peak between 30°C and 48°C and an exothermic peak between 200°C and 245°C;

(2) the oil and fat have a solid fat content curve with a slope of -2.51 to -1.57 at 10°C to 40°C;

(3) the oil and fat have a viscosity of 23.900 mPa·s to 84.445 mPa·s at 95°C;

(4) the oil and fat are selected from a vegetable oil and fat and/or an animal oil and fat;

(5) the oil and fat are subjected to at least one process step selected from the group consisting of transesterification, fractionation and hydrogenation; and

(6) a mass percentage of the oil and fat is 5% to 17% based on the mass of the microcapsule.

Preferably, a protein in the protein coating layer is one or more selected from the group consisting of casein, pea protein and whey protein; and/or, based on the mass of the microcapsule, a mass percentage of the protein coating layer to the mass of the microcapsule is 1% to 40%.

Preferably, the microcapsule comprises at least one of the following features:

(i) the wall material is formed by calcification, cross-linking and heat curing of the colloid;

(ii) the wall material has a differential scanning calorimetry curve with an endothermic peak between 190°C to 210°C and an exothermic peak between 250°C to 281°C;

(iii) the wall material has a turbidity of 1.3 to 1.5 cm$^{-1}$ at 25°C to 95°C;

(iv) the colloid comprises alginic acid and/or its salt and a gelling agent; preferably, a mass ratio of the gelling agent to alginic acid and/or its salt is (1 to 20): 1; further preferably, the gelling agent is one or more selected from the group consisting of a thermoreversible gel, curdlan gum, konjac gum and gellan gum;

(v) based on the mass of the microcapsule, a mass percentage of the colloid is 0.2% to 7%.

Preferably, the core material of the microcapsule comprises at least one bioactive substance selected from the group consisting of probiotics, vitamins, polyunsaturated fatty acids, active proteins and natural pigments.

The present disclosure further provides a method for producing a heat-resistant and shear-resistant microcapsule, comprising steps of:

S1) mixing a biologically active substance and an oil and fat to obtain a core material;

S2) mixing and emulsifying the core material with a protein emulsion to obtain a core material emulsion;

S3) mixing and stirring the core material emulsion with a wall material solution to obtain a mixed solution; wherein the wall material solution comprises a colloid; and

S4) subjecting the mixed solution to heat treatment to obtain the microcapsule.

Preferably, a mass concentration of the protein emulsion is 1% to 5%; a mass ratio of the core material to the protein emulsion is 1:(1 to 10); and/or,
a mass concentration of the colloid in the wall material solution is 0.1% to 1%; a mass ratio of the core material emulsion to the wall material solution is 1:(2 to 7); and/or, the heat treatment is performed at a temperature of 75°C to 95°C; and/or, the heat treatment has a duration of 10 to 30 min.

The present disclosure further provides use of the above-mentioned heat-resistant and shear-resistant microcapsule as a food additive.

The present disclosure further provides a food, comprising the above-mentioned heat-resistant and shear-resistant microcapsule.

The present disclosure provides a heat-resistant and shear-resistant microcapsule, comprising a core material, a protein coating layer wrapping the core material, and a wall material wrapping the protein coating layer; the core material comprises oil and fat; and the wall material is formed by heat-curing of the colloid. Compared with the prior art, the microcapsule provided by the present disclosure first uses protein to wrap the inner phase oil to provide an internal environment that isolates moisture and oxygen and enhance the viability and stability of active ingredients during heat treatment by combining phase change endothermic effects at melting point, and uses the wall material for further protection, so that the microcapsules have higher stability, high temperature resistance, strong shear resistance, and higher acid resistance.

The experimental results show that the microcapsules provided by the present disclosure are comprehensively improved by more than 1 million times in terms of high temperature shear resistance, shear resistance, and gastric acid resistance, effectively reducing the loss of probiotics during processing and gastrointestinal digestion.

**DETAILED DESCRIPTION**

The technical solutions in the examples of the present disclosure will be clearly and completely described below in conjunction with the examples of the present disclosure. Apparently, the described examples are only part of the examples of the present disclosure, not all of the examples. Based on the examples of the present disclosure, all other examples obtained by those of ordinary skills in the art without inventive work belong to the scope of protection of the present disclosure.

The present disclosure provides a high-temperature-resistant and shear-resistant colloid, and the colloid has a differential scanning calorimetry (DSC) curve with an endothermic peak between 190°C to 210°C and an exothermic peak between 270°C to 281°C.

In the present disclosure, preferably, the high-temperature-resistant and shear-resistant colloid has a differential scanning calorimetry (DSC) curve with an endothermic peak between 190°C to 201°C. In the present disclosure, preferably, the high-temperature-resistant shear-resistant colloid has a differential scanning calorimetry (DSC) curve with an exothermic peak between 256°C to 281°C; more preferably, the high-temperature-resistant and shear-resistant colloid has a differential scanning calorimetry (DSC) curve with an exothermic peak between 269°C to 281°C.

In the present disclosure, the range of the DSC is preferably 25°C to 300°C; the heating rate of the DSC is preferably 0.5 to 1°C/min; in the present disclosure, more preferably, the colloid is kept at a constant temperature of 25°C for 5 to 10 min and then heated; further preferably, the obtained DSC curve has a wide endothermic peak between 190°C to 210°C, and the width of the endothermic peak is preferably 57°C to 70°C, more preferably 60 to 70°C, and more preferably 60 to 66°C.

The turbidity of the colloid provided by the present disclosure at 25°C to 95°C is preferably 1.3 to 1.5 cm$^{-1}$, more preferably 1.32 to 1.5 cm$^{-1}$, more preferably 1.4 to 1.5 cm$^{-1}$, more preferably 1.4 to 1.48 cm$^{-1}$, and most preferably 1.4 to 1.45 cm$^{-1}$.

The colloid provided by the present disclosure preferably comprises a thermal gel, and preferably also comprises alginic acid and/or its salt; the mass ratio of the thermal gel to alginic acid and/or its salt is preferably (1-20):1, more preferably (5-20):1, more preferably (8-15):1, and most preferably (10-12):1; the thermal gel is preferably one or more selected from the group consisting of a thermoreversible gel, curdlan gum, konjac gum and gellan gum; the thermoreversible gel is preferably pectin.

The colloid is preferably obtained by heat treatment, more preferably obtained by heat treatment of an aqueous solution containing thermal gel, and more preferably obtained by heat treatment of an aqueous solution containing thermal gel and alginic acid and/or its salt; the mass concentration of the aqueous solution containing thermal gel is preferably 0.1% to 1%; the mass concentration of the aqueous solution containing thermal gel and alginic acid and/or its salt is preferably 0.1% to 1%; the temperature of the heat treatment is preferably 75°C to 95°C; the duration of the heat treatment is preferably 30 to 120 s; more preferably, calcification and cross-linking is conducted before the heat treatment; the solution of the calcification and cross-linking is preferably 1 to 20 wt% calcium salt solution; the calcium salt solution is preferably calcium chloride solution and/or calcium lactate solution; when the calcium salt solution is calcium chloride solution, its concentration is preferably 1 to 10 wt%; when the calcium salt solution is calcium lactate solution, its concentration is preferably 1 to 20 wt%, more preferably 3 to 15 wt%, more preferably 5 to 15 wt%, and most preferably 5 to 10 wt%; the duration of calcification and cross-linking is preferably 10 to 20 min.

The present disclosure further provides use of the above-mentioned colloid in improving heat resistance of a bioactive substance.

Wherein, the heat resistance is preferably the heat resistance of the bioactive substance in a shear environment; the bioactive substance is preferably a heat-sensitive substance, more preferably one or more selected from the group consisting of probiotics, vitamins, polyunsaturated fatty acids, active proteins and natural pigments.

The present disclosure also provides a food comprising the above-mentioned colloid.

The present disclosure also provides use of the above-mentioned colloid as a wall material for microcapsule.

The present disclosure also provides a microcapsule, comprising a core material and a wall material wrapping the core material; the wall material comprises the above-mentioned colloid. Based on the mass ratio of dry matter, the mass ratio of the core material to the colloid is preferably 5.56:1 to 1.59:1.

In the microcapsule provided by the present disclosure, the core material preferably comprises a bioactive substance and oil and fat; the bioactive substance is the same as described above and will not be repeated here; the oil and fat can be any oil and fat known to those skilled in the art without any special restrictions. In the present disclosure, the oil and fat is preferably a vegetable oil and fat, more preferably one or more selected from the group consisting of soybean oil, olive oil, sunflower oil and cocoa butter; the mass of the oil and fat is preferably 5% to 17% of the mass of the microcapsule.

Preferably, a protein coating layer is included between the core material and the wall material; the protein coating layer is formed by a protein aqueous solution; the type of protein in the protein coating layer can be a protein well known to those skilled in the art, and there is no special restriction. In the present disclosure, it is preferably a natural protein with strong heat resistance and gelling properties, and more preferably one or more selected from the group consisting of casein, pea protein and whey protein; the mass of the protein coating layer is preferably 6% to 30% of the mass of the microcapsule. Moreover, the ratio of the total mass of the core material and the protein coating layer to the mass of the colloid is preferably 1:2 to 1:7 based on the mass of the hydrate; in the examples provided by the present disclosure, the ratio of the total mass of the core material and the protein coating layer to the mass of the colloid is specifically 1:2, 1:7, 1:3, or 1:5.

The water concentration in the microcapsule provided by the present disclosure is preferably 75% to 90%, and more preferably 76.33% to 87.68%; for easy storage, the microcapsule is preferably dried to a water concentration of less than 1%.

The present disclosure also provides a method for producing the above-mentioned microcapsules, comprising the following steps: S1) mixing a colloidal raw material with water to obtain a colloidal raw material solution; S2) mixing and stirring the core material with the colloidal raw material solution to obtain a mixed solution; S3) subjecting the mixed solution to heat treatment to obtain the microcapsule.

Among them, the present disclosure has no special restrictions on the sources of all raw materials, and they can be commercially available.

The colloidal raw material is mixed with water to obtain a colloidal solution; the colloidal raw material preferably comprises a thermal gel, and more preferably further comprises alginic acid and/or its salt; the mass ratio of the thermal gel to alginic acid and/or its salt is preferably (1-20):1, more preferably (5-20):1, more preferably (8-15):1, and most preferably (10-12):1; the thermal gel is preferably one or more selected from the group consisting of thermoreversible gel, curdlan gum, konjac gum and gellan gum; the thermoreversible gel is preferably pectin; the alginate is a salt compound well known to those skilled in the art, and there is no special limitation, and in the present disclosure, sodium alginate is preferred; the water is preferably deionized water; the mixing temperature is preferably 30°C to 50°C; the mixing rate is preferably 200 to 1000 rpm; the mixing time is preferably 20 to 60 min, and more preferably 30 to 40 min; the mass concentration of the colloidal raw material in the colloidal solution is preferably 0.1% to 1%; in the examples provided by the present disclosure, the mass concentration of the colloidal raw material in the colloidal solution is specifically 1%, 0.1%, 0.3% or 0.5%; the above colloid can be obtained by subjecting the colloidal raw material solution to heat treatment.

The core material and the colloidal raw material solution are mixed and stirred to obtain a mixed solution; in the present disclosure, the core material is preferably prepared according to the following method: a bioactive substance is dispersed in oil and fat to obtain the core material; the bioactive substance and the oil and fat are the same as described above, and will not be repeated here; the dispersion time is preferably 2 to 4 min. The mass ratio of the core material to the colloidal solution is preferably 1:(2-7); in the examples provided by the present disclosure, the mass ratio of the core material to the colloidal solution is specifically 1:2, 1:7, 1:3, or 1:5; the temperature of the mixing and stirring is preferably 30°C-50°C; in the examples provided by the present disclosure, the temperature of the mixing and stirring is specifically 30°C, 50°C, 40°C or 45°C; the speed of the mixing and stirring is preferably 500-2000 rpm, more preferably 800-2000 rpm, and more preferably 800-1500 rpm; the duration of the mixing and stirring is preferably 20-30 s.

In the present disclosure, it is further preferred that the core material is preferably emulsified with a protein aqueous solution to obtain a core material emulsion, and then mixed and stirred with a colloidal raw material solution; the protein aqueous solution is preferably a protein aqueous solution sterilized by ultraviolet light; the mass concentration of the protein aqueous solution is preferably 1% to 5%, more preferably 2% to 4%, and more preferably 3%; the duration of the ultraviolet sterilization is preferably 10 to 20 min, more preferably 15 min; the protein aqueous solution is preferably prepared according to the following steps: mixing protein with water, adjusting to neutrality, and stirring to obtain a protein aqueous solution; the protein aqueous solution is preferably one or more selected from the group consisting of pea protein, casein, whey protein and soy protein isolate; the stirring speed is preferably 500 to 1000 rpm, more preferably 600 to 800 rpm; the stirring time is preferably 1 to 5 h, more preferably 2 to 3 h; the mass ratio of the core material emulsion to the colloidal solution is preferably 1:(2 to 7); in the examples provided by the present disclosure, the mass ratio of the core material emulsion to the colloidal solution is specifically 1:2, 1:7, 1:3, or 1:5; the temperature of the mixing and stirring is preferably 30°C to 50°C; in the examples provided by the present disclosure, the temperature of the mixing and stirring is specifically 30°C, 50°C, 40°C or 45°C; the speed of the mixing and stirring is preferably 500 to 2000 rpm, more preferably 800 to 2000 rpm, and more preferably 800 to 1500 rpm; the duration of the mixing and stirring is preferably 20 to 30 s.

The mixed solution is subjected to heat treatment, preferably subjected to calcification and cross-linking before the heat treatment, to obtain a microcapsule; the solution for calcification and cross-linking is preferably a 1-20 wt% calcium salt solution; the calcium salt solution is preferably a calcium chloride solution and/or a calcium lactate solution; when the calcium salt solution is a calcium chloride solution, its concentration is preferably 1-10 wt%; when the calcium salt solution is a calcium lactate solution, its concentration is preferably 1-20 wt%, more preferably 3-15 wt%, more preferably 5-15 wt%, and most preferably 5-10 wt%; the duration for calcification and cross-linking is preferably 10-20 min; the temperature for heat curing is preferably 75°C-95°C; in the examples provided by the present disclosure, the temperature of the heat curing is specifically 95°C, 75°C, 80°C or 85°C; the duration of the heat curing is preferably 30-120 s.

The finally obtained microcapsule is preferably stored at 4°C.

The present disclosure also provides use of the above-mentioned microcapsule as a food additive, which can be used in food processing.

The present disclosure also provides use of the above-mentioned microcapsule in improving heat resistance of a bioactive substance; preferably, the heat resistance is the heat resistance of the bioactive substance in a shear environment; the bioactive substance is a heat-sensitive substance.

The present disclosure also provides a food comprising the above-mentioned microcapsule.

The present disclosure also provides a heat-resistant and shear-resistant colloidal wall material, which has a differential scanning calorimetry (DSC) with an endothermic peak between 190°C and 210°C and an exothermic peak between 250°C and 281°C.

In the present disclosure, preferably, the differential scanning calorimetry (DSC) curve of the colloidal wall material has an endothermic peak between 190°C and 201°C. In the present disclosure, preferably, the differential scanning calorimetry (DSC) curve of the colloidal wall material has an exothermic peak between 256°C and 281°C; more preferably, the differential scanning calorimetry (DSC) curve of the colloidal wall material has an exothermic peak between 269°C and 281°C.

In the present disclosure, the range of the DSC is preferably 25°C to 300°C; the heating rate of the DSC is preferably 0.5 to 1°C/min; in the present disclosure, more preferably, the colloidal wall material is kept at a constant temperature of 25°C for 5 to 10 min and then heated; the obtained DSC curve has a wide endothermic peak between 190°C and 210°C; the width of the endothermic peak is preferably 57°C to 70°C, more preferably 60°C to 70°C.

The turbidity of the colloidal wall material provided by the present disclosure at 25°C to 95°C is preferably 1.3 to 1.5 cm$^{-1}$, more preferably 1.32 to 1.5 cm$^{-1}$, more preferably 1.4 to 1.5 cm$^{-1}$, more preferably 1.4 to 1.48 cm$^{-1}$, and most preferably 1.4 to 1.45 cm$^{-1}$.

The colloidal wall material provided by the present disclosure is preferably formed by heat curing the colloid, and more preferably formed by subjecting the colloid to calcification, cross-linking and heat curing; the colloid preferably comprises alginic acid and/or its salt and a gelling agent; the mass ratio of the gelling agent to sodium alginate and/or its salt is preferably (1-20):1, more preferably (5-20):1, more preferably (8-15):1, and most preferably (10-12):1; the alginate may be an alginate known to those skilled in the art, and there is no special restriction, and sodium alginate is preferably used in the present disclosure; the gelling agent is preferably one or more selected from the group consisting of a thermoreversible gel, curdlan gum, konjac gum and gellan gum; the thermoreversible gel is preferably pectin.

The present disclosure also provides use of the above-mentioned colloidal wall material as a microcapsule wall material.

The present disclosure also provides a heat-resistant and acid-resistant oil and fat, which has a differential scanning calorimetry curve with an endothermic peak between 30°C and 50°C and an exothermic peak between 200°C and 245°C.

Preferably, the differential scanning calorimetry curve of the oil and fat has an endothermic peak between 30°C and 48°C, and an exothermic peak between 200°C and 241°C.

The oil and fat has a solid fat content curve with a slope of preferably -2.51 to -1.57 and more preferably -2.45 to -1.85 at 10°C to 40°C.

The viscosity value of the oil and fat at 95°C is preferably 23.900 mPa·s to 84.445 mPa·s; in the examples provided by the present disclosure, the viscosity value of the oil at 95°C is specifically 25.2 mPa·s or 28 mPa·s.

In the present disclosure, the oil and fat is preferably a vegetable oil and fat and/or an animal oil and fat.

In the present disclosure, the oil and fat is preferably subjected to at least one process step including transesterification, fractionation and hydrogenation.

The oil and fat with the above-mentioned specific parameters provided by the present disclosure preferably comprises one or more of a mixed oil, a mixed oil after transesterification, an intermediate fractionation oil and a hydrogenated oil.

Wherein, the mixed oil preferably comprises unsaturated fatty acid oil and saturated fatty acid oil; the content of unsaturated fatty acids in the unsaturated fatty acid oil is greater than 50%; the content of saturated fatty acids in the saturated fatty acid oil is greater than 50%; in the present disclosure, the unsaturated fatty acid oil is preferably one or more selected from the group consisting of palm kernel oil, rapeseed oil, rice bran oil, soybean oil, sunflower seed oil, rapeseed oil and corn oil; the saturated fatty acid oil is one or more selected from the group consisting of coconut oil, palm stearin, butter, coconut oil and cocoa butter; the mass ratio of unsaturated fatty acid oil to saturated fatty acid oil in the mixed oil is preferably (10 to 70): (90 to 30); in the present disclosure, it is further preferred that when the oil and fat composition comprises the mixed oil, in the mixed oil, the mass ratio of the unsaturated fatty acid oil to saturated fatty acid oil is (20-50): (80-50), more preferably (20-40): (80-60), and more preferably (20-30): (80-70); when the oil and fat composition includes the mixed oil after transesterification, the mass ratio of unsaturated fatty acid oil to saturated fatty acid oil in the mixed oil is (10-70): (80-30); the catalyst used in the esterification treatment is a catalyst well known to those skilled in the art, and there is no special limitation. In the present disclosure, lipase and/or sodium methoxide are preferably used; the mass of the catalyst is preferably 1%-3% of the mass of the mixed oil, more preferably 1%; the temperature of the esterification treatment is preferably 35°C-130°C; the duration of the esterification treatment is preferably 0.5-4 h; the intermediate fractionated oil is preferably the intermediate fractionated oil of palm oil, more preferably 14-44 degree palm oil; in the examples provided by the present disclosure, the intermediate fractionated oil is specifically 44 degree palm oil, 33 degree palm oil, 24 degree palm oil or 14 degree palm oil; the hydrogenated oil is preferably hydrogenated vegetable oil, more preferably one or more selected from the group consisting of hydrogenated soybean oil, hydrogenated peanut oil, hydrogenated flaxseed oil and hydrogenated rapeseed oil; the hydrogenated oil is obtained by hydrogenating oil; the reaction pressure of the hydrogenation is preferably 0.02-0.8 MPa, more preferably 0.02-0.5 MPa; in the examples provided by the present disclosure, the reaction pressure of the hydrogenation is specifically 0.02 MPa, 0.5 MPa, 0.1 MPa or 0.3 MPa; the reaction temperature of the hydrogenation is preferably 100°C-180°C; in the examples provided by the present disclosure, the reaction temperature of the hydrogenation is specifically 100°C, 180°C, 130°C or 150°C; the reaction time of the hydrogenation is preferably 2-4 h.

More specifically, in the examples provided by the present disclosure, shortening is used as the oil and fat; further specifically, the shortening is derived from palm oil or cocoa butter.

The present disclosure also provides use of the above-mentioned oil and fat in encapsulating a bioactive substance.

The present disclosure also provides a heat-resistant and acid-resistant oil and fat composition, which has a differential scanning calorimetry curve with an endothermic peak between 35°C and 47°C and an exothermic peak between 220°C and 250°C;

The slope of the solid fat content curve of the oil and fat composition at 20 to 50°C is -3.0 to -1.0, preferably -2.82 to -1.2.

In the examples provided by the present disclosure, the slope of the solid fat content curve of the oil and fat composition at 20 to 50°C is specifically -2.86, -1.22, -2.45, -1.76, -2.61, -1.6, -2.22, -1.87, -2.82, -1.18, -2.66 or -1.73.

According to the present disclosure, the viscosity of the oil and fat composition at 95°C is preferably 20-90 mPa·s, more preferably 25-86 mPa·s.

The oil and fat composition with the above-mentioned specific parameters provided by the present disclosure preferably comprises one or more selected from the group consisting of a mixed oil, a mixed oil after transesterification, an intermediate fractionation oil and a hydrogenated oil.

Wherein, the mixed oil preferably includes unsaturated fatty acid oil and saturated fatty acid oil; the content of unsaturated fatty acids in the unsaturated fatty acid oil is greater than 50%; the content of saturated fatty acids in the saturated fatty acid oil is greater than 50%; in the present disclosure, the unsaturated fatty acid oil is preferably one or more selected from the group consisting of palm kernel oil, rapeseed oil, rice bran oil, soybean oil, sunflower seed oil, rapeseed oil and corn oil; the saturated fatty acid oil is one or more selected from the group consisting of coconut oil, palm stearin, butter, coconut oil and cocoa butter; the mass ratio of unsaturated fatty acid oil to saturated fatty acid oil in the mixed oil is preferably (10 to 70): (90 to 30); in the present disclosure, it is further preferred that when the oil and fat composition comprises the mixed oil, in the mixed oil, the mass ratio of the unsaturated fatty acid oil to saturated fatty acid oil is (20-50): (80-50), more preferably (20-40): (80-60), and more preferably (20-30): (80-70); in the examples provided by the present disclosure, the mass ratio of unsaturated fatty acids to saturated fatty acids in the mixed oil is specifically 30:70, 60:40, 75:25 or 50:50; when the oil and fat composition comprises the mixed oil after transesterification, the mass ratio of unsaturated fatty acid oil to saturated fatty acid oil in the mixed oil is (10-70): (80-30); the catalyst used in the esterification treatment is a catalyst well known to those skilled in the art, and there is no special limitation. In the present disclosure, lipase and/or sodium methoxide are preferably used; the mass of the catalyst is preferably 1%-3% of the mass of the mixed oil, more preferably 1%; the temperature of the esterification treatment is preferably 35°C-130°C; the duration of the esterification treatment is preferably 0.5-4 h; the intermediate fractionated oil is preferably the intermediate fractionated oil of palm oil, more preferably 14-44 degree palm oil; in the examples provided by the present disclosure, the intermediate fractionated oil is specifically 44 degree palm oil, 33 degree palm oil, 24 degree palm oil or 14 degree palm oil; the hydrogenated oil is preferably hydrogenated vegetable oil, more preferably one or more selected from the group consisting of hydrogenated soybean oil, hydrogenated peanut oil, hydrogenated flaxseed oil and hydrogenated rapeseed oil; the hydrogenated oil is obtained by hydrogenating oil; the reaction pressure of the hydrogenation is preferably 0.02-0.8 MPa, more preferably 0.02-0.5 MPa; in the examples provided by the present disclosure, the reaction pressure of the hydrogenation is specifically 0.02 MPa, 0.5 MPa, 0.1 MPa or 0.3 MPa; the reaction temperature of the hydrogenation is preferably 100°C-180°C; in the examples provided by the present disclosure, the reaction temperature of the hydrogenation is specifically 100°C, 180°C, 130°C or 150°C; the reaction time of the hydrogenation is preferably 2-4 h.

The present disclosure also provides use of the above-mentioned oil and fat composition in encapsulating a bioactive substance.

The present disclosure also provides a food comprising the above-mentioned oil and fat composition.

The present disclosure also provides a microcapsule, comprising a core material and a wall material wrapping the core material; wherein the core material comprises the above-mentioned oil and fat composition.

According to the present disclosure, the mass of the oil and fat composition is preferably 5% to 17% of the mass of the microcapsule; the core material preferably also comprises a bioactive substance; the bioactive substance is a bioactive substance well known to those skilled in the art, without any special restrictions, and in the present disclosure, it is preferably a bioactive substance that is unstable to heat, and more preferably one or more selected from the group consisting of probiotics, vitamins, DHA, lactoferrin and anthocyanins.

According to the present disclosure, a protein coating layer is preferably provided between the core material and the wall material; the type of protein in the protein coating layer is a protein well known to those skilled in the art, without any special restrictions, and in the present disclosure, it is preferably a natural protein with strong heat resistance and gelling properties, and more preferably one or more selected from the group consisting of casein, pea protein and whey protein; the mass of the protein coating layer is preferably 6% to 30% of the mass of the microcapsule.

According to the present disclosure, the wall material is preferably formed by heat curing of the colloid, more preferably by calcification, cross-linking and heat curing; the wall material has a differential scanning calorimetry curve with an endothermic peak between 190°C and 210°C and an exothermic peak between 270°C and 280°C; the DSC range is preferably 25°C to 300°C; the heating rate of the DSC is preferably 0.5 to 1°C/min; in the present disclosure, more preferably, the colloid is kept at a constant temperature of 25°C for 5 to 10 min and then heated; in the present disclosure, the obtained DSC curve preferably has a wide endothermic peak between 190°C and 210°C, and the width of the endothermic peak is preferably 57°C to 70°C, more preferably 60°C to 70°C; the turbidity of the wall material at 25°C to 95°C is preferably 1.3 to 1.5 cm$^{-1}$, more preferably 1.32 to 1.5 cm$^{-1}$, still more preferably 1.4 to 1.5 cm$^{-1}$, and even more preferably 1.4 to 1.48 cm$^{-1}$, and most preferably 1.4 to 1.45 cm$^{-1}$. The colloid provided by the present disclosure preferably comprises alginic acid and/or its salt and a gelling agent; the mass ratio of the gelling agent to sodium alginate and/or its salt is preferably (1 to 20):1, more preferably (5 to 20):1, still more preferably (8 to 15):1, and most preferably (10 to 12):1; the gelling agent is preferably one or more selected from the group consisting of a thermoreversible gel, curdlan gum, konjac gum and gellan gum; the thermoreversible gel is preferably pectin. Based on the mass ratio of dry matter, the mass ratio of the core material to the colloid is preferably 5.56:1 to 1.59:1; based on the mass of hydrates, the mass ratio of the total mass of the core material and the protein coating layer to the mass of the colloid is preferably 1:2 to 1:7; in the examples provided by the present disclosure, the mass ratio of the total mass of the core material and the protein coating layer to the mass of the colloid is specifically 1:2, 1:7, 1:3, or 1:5.

The water concentration in the microcapsules provided by the present disclosure is preferably 75% to 90%, more preferably 76.33% to 87.68%; for easy storage, it is preferable that drying is performed to a water concentration of less than 1% in the microcapsules.

The present disclosure also provides a method for producing the above-mentioned microcapsules, comprising the following steps: S1) dispersing a bioactive substance in the above-mentioned oil and fat composition to obtain a core material; S2) mixing and stirring the core material and a wall material solution to obtain a mixed solution; S3) heat curing the mixed solution to obtain the microcapsules.

Among them, the present disclosure has no special restrictions on the source of all raw materials, which can be commercially available.

The bioactive substance is dispersed in the oil and fat composition to obtain a core material; the dispersion time is preferably 2 to 4 min.

In the present disclosure, preferably, the core material is mixed with a protein emulsion and emulsified to obtain a core material emulsion; the protein emulsion is a protein aqueous solution; the protein emulsion is preferably a protein emulsion sterilized by ultraviolet light; the mass concentration of the protein emulsion is preferably 1% to 5%, more preferably 2% to 4%, and still more preferably 3%; the ultraviolet sterilization time is preferably 10 to 20 min, more preferably 15 min; the protein emulsion is preferably prepared according to the following steps: mixing protein with water, adjusting to neutrality, and stirring to obtain a protein emulsion; the type of protein in the protein emulsion is the same as described above and will not be repeated here; the stirring speed is preferably 500 to 1000 rpm, more preferably 600 to 800 rpm; the stirring time is preferably 1 to 5 h, more preferably 2 to 3 h; the mass ratio of the core material to the protein emulsion is preferably 1: (1-10), more preferably 1: (1-8), still more preferably 1: (1-5), even more preferably 1: (1-4), and most preferably 1: (2-3); the rotation speed of the mixing and emulsification is preferably 10000-20000 rpm, more preferably 11000-15000 rpm, still more preferably 11000-13000 rpm, and most preferably 12000 rpm; and the time of the mixing and emulsification is preferably 3-5 min.

Then the core material emulsion is mixed and stirred with the wall material solution to obtain a mixed solution; the wall material solution is preferably prepared according to the following method: a colloid is mixed with water to obtain the wall material solution; the type of the colloid is the same as described above and will not be repeated here; the water is preferably deionized water; the mixing temperature is preferably 30°C to 50°C; the mixing rate is preferably 200 to 1000 rpm; the mixing time is preferably 20 to 60 min, more preferably 30 to 40 min; the mass concentration of the colloid in the wall material solution is preferably 0.1% to 1%; in the examples provided by the present disclosure, the mass concentration of the colloid in the wall material solution is specifically 1%, 0.1%, 0.3% or 0.5%; the mass ratio of the core material emulsion to the wall material solution is preferably 1:(2 to 7); in the examples provided by the present disclosure, the mass ratio of the core material emulsion to the wall material solution is specifically 1:2, 1:6, 1:7, 1:3, or 1:5; the mixing and stirring temperature is preferably 30°C to 50°C; in the examples provided by the present disclosure, the mixing and stirring temperature is specifically 30°C, 50°C, 40°C or 45°C; the mixing and stirring speed is preferably 500 to 2000 rpm, more preferably 800 to 2000 rpm, and still more preferably 800-1500 rpm; the mixing and stirring time is preferably 20-30 s.

The mixed solution is subjected to heat curing, preferably calcification cross-linking and heat curing to obtain microcapsules; the solution for calcification and cross-linking is preferably a 1-20 wt% calcium salt solution; the calcium salt solution is preferably a calcium chloride solution and/or a calcium lactate solution; when the calcium salt solution is a calcium chloride solution, its concentration is preferably 1-10 wt%; when the calcium salt solution is a calcium lactate solution, its concentration is preferably 1-20 wt%, more preferably 3-15 wt%, still more preferably 5-15 wt%, and most preferably 5-10 wt%; the time for calcification and cross-linking is preferably 10-20 min; the temperature for heat curing is preferably 75°C-95°C; in the examples provided by the present disclosure, the temperature for heat curing is specifically 95°C, 75°C, 80°C or 85°C; the time for heat curing is preferably 30-120 s, more preferably 1 min. In this step, the colloidal substance forms microcapsules under the action of calcium bridges to achieve encapsulation and shaping, and initially has certain shear resistance, high temperature resistance and digestion resistance. Then, the microcapsules are further cured by heating to form irreversible, stable microcapsules with high temperature resistance and shear resistance.

The microcapsules finally obtained are preferably stored at 4°C.

In the microcapsules provided by the present disclosure, the bioactive substance is dispersed in the oil and fat composition, and the protein is used for encapsulation, which provides an internal environment that isolates moisture and oxygen, and combines the melting point phase change to absorb heat to improve the survival rate of strains during heat treatment; the amphiphilicity of the protein makes it a natural emulsifier, forming a dense network structure to stabilize the oil-water interface, and at the same time contributes to gelation. In the formation of the microcapsule, the protein is further cross-linked with the outer polysaccharide (gel) substance, so that the entire microcapsule is more solid and stable, and the environmental stability of the bioactive substance is improved.

The present disclosure also provides use of the above-mentioned microcapsules as a food additive, which can be used in food processing.

The present disclosure also provides a food, comprising the above-mentioned microcapsules.

The present disclosure also provides a microcapsule, comprising a core material, a protein coating layer wrapping the core material, and a wall material wrapping the protein coating layer; the core material comprises oil and fat; and the wall material is formed by heat curing of a colloid.

In the present disclosure, the core material comprises a bioactive substance and oil and fat; the bioactive substance is preferably one or more selected from the group consisting of probiotics, vitamins, polyunsaturated fatty acids, active proteins, and natural pigments; the oil and fat is the above-mentioned heat-resistant and acid-resistant oil and fat, which will not be repeated here; the mass of the oil and fat is preferably 5% to 17% of the mass of the microcapsule.

The core material is wrapped by a protein coating layer; the protein coating layer is formed by a protein; the type of the protein in the protein coating layer can be a protein well known to those skilled in the art, and there is no special restriction; in the present disclosure, it is preferably a natural protein with strong heat resistance and gelling properties, and more preferably one or more selected from the group consisting of casein, pea protein, and whey protein; the mass of the protein coating layer is preferably 1% to 40% of the mass of the microcapsule, more preferably 6% to 30%, still more preferably 6% to 20%, and most preferably 6% to 10%.

The protein coating layer is wrapped by a wall material; the wall material is formed by heat curing of a colloid; the wall material has a differential scanning calorimetry (DSC) curve with an endothermic peak between 85°C and 110°C and an exothermic peak between 270°C and 280°C. In the present disclosure, the DSC range is preferably 25°C to 300°C; the heating rate of the DSC is preferably 0.5 to 1°C/min; in the present disclosure, more preferably, the wall material is kept at a constant temperature of 25°C for 5 to 10 minutes and then heated; the resulting DSC curve has a wide endothermic peak between 85°C and 110°C; the width of the endothermic peak is preferably 57°C to 70°C, and more preferably 60°C to 70°C. The turbidity of the wall material provided by the present disclosure at 25°C to 95°C is preferably 1.3 to 1.5 cm$^{-1}$, more preferably 1.32 to 1.5 cm$^{-1}$, still more preferably 1.4 to 1.5 cm$^{-1}$, even more preferably 1.4 to 1.48 cm$^{-1}$, and most preferably 1.4 to 1.45 cm$^{-1}$. In the present disclosure, the wall material is preferably formed by heat curing of the colloid, and more preferably formed by calcification, cross-linking and heat curing of the colloid; the colloid preferably comprises alginic acid and/or its salt and a gelling agent; the mass ratio of the gelling agent to sodium alginate is preferably 10 to 12; the alginate is an alginate well known to those skilled in the art, and there is no special limitation. In the present disclosure, sodium alginate is preferably used; the gelling agent is preferably one or more selected from the group consisting of a thermoreversible gel, curdlan gum, konjac gum and gellan gum; the thermoreversible gel is preferably pectin. Based on the mass ratio of dry matter, the mass ratio of the core material to the colloid is preferably 5.56:1 to 1.59:1; based on the mass of hydrates, the mass ratio of the total mass of the core material and the protein coating layer to the colloid is preferably 1:2 to 1:7; in the examples provided by the present disclosure, the mass ratio of the total mass of the core material and the protein coating layer to the colloid is specifically 1:2, 1:7, 1:3, or 1:5.

The water concentration in the microcapsule provided by the present disclosure is preferably 75% to 90%, more preferably 76.33% to 87.68%; for easy storage, it is preferable that drying is performed to a water concentration of less than 1% in the microcapsule.

The present disclosure also provides a method for producing the above-mentioned microcapsules, comprising the following steps: S1) mixing a bioactive substance and an oil and fat to obtain a core material; S2) mixing and emulsifying the core material with a protein emulsion to obtain a core material emulsion; S3) mixing and stirring the core material emulsion with a wall material solution to obtain a mixed solution; the wall material solution comprises a colloid; S4) subjecting the mixed solution to heat treatment to obtain microcapsules.

Among them, the present disclosure has no special restrictions on the sources of all raw materials, and they can be commercially available.

The bioactive substance and the oil and fat are mixed to obtain the core material; the mixing time is preferably 2 to 4 min.

The core material is mixed and emulsified with the protein emulsion to obtain the core material emulsion; the protein emulsion solution is preferably sterilized by ultraviolet light before being mixed and emulsified with the core material; the mass concentration of the protein emulsion solution is preferably 1% to 5%, more preferably 2% to 4%, and still more preferably 3%; the ultraviolet sterilization time is preferably 10 to 20 min, more preferably 15 min; the protein emulsion is preferably prepared according to the following steps: the protein is mixed with water, adjusted to neutrality, and stirred to obtain a protein emulsion; the protein is preferably one or more selected from the group consisting of pea protein, casein, whey protein and soy protein isolate; the stirring speed is preferably 500 to 1000 rpm, more preferably 600 to 800 rpm; the stirring time is preferably 1 to 5 h, more preferably 2 to 3 h; the mass ratio of the core material to the protein emulsion is preferably 1: (1-10), more preferably 1: (1-8), still more preferably 1: (1-5), even more preferably 1: (1-4), and most preferably 1: (2-3); the rotation speed of the mixed emulsification is preferably 10000-20000 rpm, more preferably 11000-15000 rpm, still more preferably 11000-13000 rpm, and most preferably 12000 rpm; the time of the mixing and emulsification is preferably 3-5 min.

The core material emulsion and the wall material solution are mixed and stirred to obtain a mixed solution; the wall material solution is a colloidal aqueous solution, preferably prepared by the following method: mixing a colloid with water to obtain a wall material solution; the colloid preferably comprises alginic acid and/or its salt and a gelling agent; the mass ratio of the gelling agent to sodium alginate is preferably (1-20):1, more preferably (5-20):1, still more preferably (8-15):1, and most preferably (10-12):1; the alginate can be an alginate known to those skilled in the art without any special restrictions, and is preferably sodium alginate in the present disclosure; the gelling agent is preferably one or more selected from the group consisting of a thermoreversible gel, curdlan gum, konjac gum and gellan gum; the thermoreversible gel is preferably pectin; the water is preferably deionized water; the mixing temperature is preferably 30°C-50°C; the mixing rate is preferably 200-1000 rpm; the mixing time is preferably 20-60 min, more preferably 30-40 min; the mass concentration of the colloid in the wall material solution is preferably 0.1% to 1%; in the examples provided by the present disclosure, the mass concentration of the colloid in the wall material solution is specifically 1%, 0.1%, 0.3% or 0.5%.

The mass ratio of the core material emulsion to the wall material solution is preferably 1:(2 to 7); in the examples provided by the present disclosure, the mass ratio of the core material to the colloid solution is specifically 1:2, 1:7, 1:3, or 1:5; the mixing and stirring temperature is preferably 30°C to 50°C; in the examples provided by the present disclosure, the mixing and stirring temperature is specifically 30°C, 50°C, 40°C or 45°C; the rotation speed of the mixing and stirring is preferably 500 to 2000 rpm, more preferably 800 to 2000 rpm, and still more preferably 800 to 1500 rpm; the mixing time is preferably 20 to 30 s.

The mixed solution is subjected to heat treatment, preferably calcification, cross-linking and heat treatment to obtain microcapsules; the solution for calcification and cross-linking is preferably a 1-20 wt% calcium salt solution; the calcium salt solution is preferably a calcium chloride solution and/or a calcium lactate solution; when the calcium salt solution is a calcium chloride solution, its concentration is preferably 1-10 wt%; when the calcium salt solution is a calcium lactate solution, its concentration is preferably 1-20 wt%, more preferably 3-15 wt%, still more preferably 5-15 wt%, and most preferably 5-10 wt%; the time for calcification and cross-linking is preferably 10-20 min; the temperature for heat curing is preferably 75°C-95°C; in the examples provided by the present disclosure, the temperature for heat curing is specifically 95°C, 75°C, 80°C or 85°C; the time for heat curing is preferably 30-120 s.

The microcapsules finally obtained are preferably stored at 4°C.

The present disclosure also provides use of the above-mentioned microcapsules as a food additive, which can be used in food processing.

The present disclosure also provides a food comprising the above-mentioned heat-resistant and shear-resistant microcapsules.

In order to further illustrate the present disclosure, the following is a detailed description of a heat-resistant and shear-resistant microcapsule provided by the present disclosure, its production method and use in conjunction with examples.

The reagents used in the following examples are all commercially available. Oils and fats were purchased from Cargill, COFCO and Yihai Kerry; casein, pea protein, whey protein and soy protein isolate were purchased from Shengxi Biotechnology (Shanghai) Co., Ltd.; curdlan gum, pectin, konjac gum, etc. were purchased from Weifang Tianjiaji Economic and Trade Co., Ltd.; other reagents were purchased from Sinopharm. The type of bacterial powder used in the examples and comparative examples was the self-owned strain BL-99, which was deposited in the China General Microbiological Culture Collection Center (CGMCC) (address: No. 3, No. 1 Beichen West Road, Chaoyang District, Beijing, Institute of Microbiology, Chinese Academy of Sciences) on April 26, 2018, and is classified and named: *Bifidobacterium lactis;* the deposit number is CGMCC No.15650, and it has been disclosed in the applicant's prior patent application CN201811161108.3.

DSC test method:

$3.0\pm0.5$ mg of sample was weighed and placed in an aluminum box, the sample was sealed, kept at a constant temperature of 25°C for 5 min, and then heated to 300°C at a heating rate of 0.5°C/min, and the scan temperature range was 25 to 300°C.

Viscosity:

Refer to GB/T 10247-2008.

Melting curve:

Refer to GB/T 31743-2015.

Turbidity test method:

A colloidal solution dispersion was prepared, stirred at room temperature, dispersed evenly, and cooled to 25°C. 1.5 mL of the colloidal solution dispersion was removed to a preheated cuvette, the wavelength was set to 600 nm, distilled water was set as the reference solution, measurement was carried out at 600 nm on a UV spectrophotometer, and each sample was measured 3 times in parallel. Turbidity (T) is defined as:

$$T = -\ln(I/I_0)/L$$

Where: I is the transmitted light intensity, I0 is the incident light intensity, and L is the optical path length (the cuvette width in this method, i.e. 1 cm).

Examples 1-4 and Comparative Examples 1-2

Pea protein was weighed and dissolved in 100 mL of deionized water, the pH was adjusted to 7, and stirring was performed at 800 rpm for 3 h to prepare a 3% protein aqueous solution, which was sterilized with ultraviolet light for 15 min; bacterial powder (self-produced strain BL99) was stirred and dispersed in soybean oil (mass ratio 1:2), stirred for 2-4 min, and the oil and fat dispersed with bacterial powder and the protein aqueous solution were emulsified at a mass ratio of 1:2 at 12000 rpm for 4 min to obtain a core material emulsion; sodium alginate powder and curdlan gum (1:10, w/w) were mixed and evenly dispersed in deionized water to obtain a mixed colloid solution, and the specific preparation parameters are shown in Table 1; the core material emulsion and the outer layer mixed colloid solution were magnetically stirred at a mass ratio of 1:6 at 40°C and 1500 rpm for 20 s to obtain a mixed solution, which was dropped into a 10% calcium chloride solution (pH 7) and reacted for 10 min to form microcapsules; the prepared microcapsules were placed in a 95°C water bath and heated for 1 min to cure into irreversible microcapsules, which were stored at 4°C.

Table 1 Preparation parameters of Examples 1 to 4 and Comparative Examples 1 to 2

| Number | mixed colloid solution concentration (%) | Stirring temperature (°C) | Stirring speed (rpm) |
|---|---|---|---|
| Example 1 | 1 | 50 | 1000 |
| Example 2 | 0.1 | 30 | 200 |
| Example 3 | 0.3 | 40 | 400 |
| Example 4 | 0.5 | 45 | 800 |
| Comparative Example 1 | 1.5 | 55 | 1200 |
| Comparative Example 2 | 0.05 | 20 | 100 |

The mixed colloid solutions in Examples 1-4 and Comparative Examples 1-2 were dropped into a 10% calcium chloride solution (pH 7), reacted for 10 min, and then placed in a 95°C water bath and heated for 1 min for curing. The properties of the cured colloid were measured, and the results are shown in Table 2.

Table 2 Performance test results of the cured colloid in Examples 1-4 and Comparative Examples 1-2

| Number | Colloidal DSC endothermic peak | Colloidal DSC exothermic peak | turbidity ($cm^{-1}$) at 25°C to 95°C | Width of endothermic peak |
|---|---|---|---|---|
| Example 1 | 202.48 | 280.88 | 1.45 | 60.95 |
| Example 2 | 190.10 | 269.24 | 1.40 | 64.82 |
| Example 3 | 192.57 | 271.76 | 1.41 | 62.04 |
| Example 4 | 198.36 | 278.40 | 1.42 | 65.51 |
| Comparative Example 1 | 218.19 | 290.99 | 1.50 | 52.89 |
| Comparative Example 2 | 182.24 | 240.27 | 1.35 | 50.16 |

Examples 5-8 and Comparative Examples 3-4

Pea protein was weighed and dissolved in 100 mL of deionized water, the pH was adjusted to 7, and stirring was performed at 800 rpm for 3 h to prepare a 3% protein aqueous solution, which was sterilized with ultraviolet light for 15 min; bacterial powder (self-produced strain BL99) was stirred and dispersed in soybean oil (mass ratio 1:2), stirred for 2-4 min, and the oil and fat dispersed with bacterial powder and the protein aqueous solution were emulsified at 12000 rpm for 4 min in a mass ratio of 1:2 to obtain a core material emulsion; sodium alginate powder and curdlan gum (1:10, w/w) were mixed and evenly dispersed in deionized water, and stirred at 800 rpm for 30 min at 45°C to obtain a mixed colloid solution with a colloidal raw material mass concentration of 0.5%; the core material emulsion and the outer layer mixed colloid solution were magnetically stirred for 20 s to obtain a mixed solution, which was dropped into a 10% calcium chloride solution (pH 7) and reacted for 10 min to form microcapsules. The specific parameters are shown in Table 3; the prepared microcapsules were placed in a 95°C water bath and heated for 1 min to cure into irreversible microcapsules, which were stored at 4°C.

Table 3 Preparation parameters of Examples 5 to 8 and Comparative Examples 3 to 4

| Number | Core material emulsion: outer layer colloid solution | Stirring temperature (°C) | Stirring speed (rpm) |
|---|---|---|---|
| Example 5 | 1:2 | 30 | 500 |
| Example 6 | 1:7 | 50 | 2000 |
| Example 7 | 1:3 | 40 | 1500 |
| Example 8 | 1:5 | 45 | 800 |
| Comparative Example 3 | 1:10 | 55 | 1200 |
| Comparative Example 4 | 1:1 | 20 | 200 |

The outer layer colloid solution in Examples 5-8 and Comparative Examples 3-4 was dropped into a 10% calcium chloride solution (pH 7), reacted for 10 min, and then placed in a 95°C water bath and heated for 1 min for curing, and the properties of the cured colloid were measured. The results were basically consistent with those in Example 4.

**Examples 9-12 and Comparative Examples 5-6**

Pea protein was weighed and dissolved in 100 mL of deionized water, the pH was adjusted to 7, and stirring was performed at 800 rpm for 3 h to prepare a 3% protein aqueous solution, which was sterilized with ultraviolet light for 15 min; the bacterial powder (self-produced strain BL99) was stirred and dispersed in soybean oil (mass ratio 1:2), stirred for 2-4 min, and the oil and fat dispersed with bacterial powder and the protein aqueous solution were emulsified at a mass ratio of 1:2 at 12000 rpm for 4 min to obtain a core material emulsion; sodium alginate powder and curdlan gum (1:10, w/w) were mixed and evenly dispersed in deionized water, and stirred at 400 rpm and 40°C for 30 min to obtain a mixed colloid solution with a colloidal raw material mass concentration of 0.5%; the core material emulsion and the outer layer mixed colloid solution were magnetically stirred at a mass ratio of 1:6 at 40°C and 1500 rpm for 20 s to obtain a mixed solution, which was then dropped in 10% calcium chloride solution (pH 7), and reacted for 10 minutes to form microcapsules; the prepared microcapsules were placed in a water bath and heated for 1 minute to cure into irreversible microcapsules, which were stored at 4°C. The water bath temperature is shown in Table 4.

The mixed colloid solution in Examples 9 to 12 and Comparative Examples 5 to 6 was dropped into a 10% calcium chloride solution (pH 7), reacted for 10 minutes, and then placed in a water bath and heated for 1 minute for curing. The properties of the cured colloid were measured. The water bath temperature is shown in Table 4, and the results are shown in Table 5.

Table 4 Preparation parameters of Examples 9 to 12 and Comparative Examples 5 to 6

| Number | Curing temperature (°C) |
|---|---|
| Example 9 | 95 |
| Example 10 | 75 |
| Example 11 | 80 |
| Example 12 | 85 |
| Comparative Example 5 | 70 |
| Comparative Example 6 | 100 |

Table 5 Performance test results of mixed colloid solutions after curing of Examples 9-12 and Comparative Examples 5-6

| Number | Colloidal DSC endothermic peak | Colloidal DSC exothermic peak | Turbidity at 25°C to 95°C | Width of endothermic peak |
|---|---|---|---|---|
| Example 9 | 198.92 | 277.15 | 1.42 | 62.04 |
| Example 10 | 191.10 | 272.40 | 1.41 | 60.95 |
| Example 11 | 193.84 | 278.83 | 1.42 | 64.82 |
| Example 12 | 193.36 | 274.72 | 1.45 | 65.51 |
| Comparative Example 5 | 186.19 | 237.92 | 1.32 | 53.19 |
| Comparative Example 6 | 201.24 | 284.37 | 1.33 | 52.64 |

Comparative Example 7

Pea protein was weighed and dissolved in 100 mL of deionized water, the pH was adjusted to 7, and stirring was performed at 800 rpm for 3 h to prepare a 3% protein aqueous solution, which was sterilized with ultraviolet light for 15 min; the bacterial powder (self-produced strain BL99) was stirred and dispersed in soybean oil (mass ratio 1:2), and stirred for 2-4 min. The oil and fat dispersed with bacterial powder and the protein aqueous solution were emulsified at a mass ratio of 1:2 at 12000 rpm for 4 min to obtain a core material emulsion; sodium alginate powder and gellan gum (1:10, w/w) were mixed and evenly dispersed in deionized water, and stirred at 400 rpm at 40°C for 30 min to obtain a mixed colloid solution with a colloidal raw material mass concentration of 0.5%; the core material emulsion and the outer layer mixed colloid solution were magnetically stirred at 40°C and 1500 rpm for 20 s at a mass ratio of 1:6 to obtain a mixed solution, which was dropped into a 10% calcium chloride solution (pH 7) and reacted for 10 min to form microcapsules; the prepared microcapsules were placed in a 70°C water bath and heated for 1 min to cure into irreversible microcapsules, which were stored at 4°C.

The mixed colloid solution in comparative example 7 was dropped into a 10% calcium chloride solution (pH 7) and reacted for 10 min to form microcapsules; the prepared microcapsules were placed in a 70°C water bath and heated for 1 min for curing, and the performance of the cured colloid was tested. After testing, the DSC endothermic peak of the colloid was at 182.65°C, the width of the endothermic peak was 54.72, the exothermic peak was at 250.30°C, and the turbidity at 25°C to 95°C was 1.36 cm$^{-1}$.

Example 13

Pea protein was weighed and dissolved in 100 mL of deionized water, the pH was adjusted to 7, and stirring was performed at 800 rpm for 3 h to prepare a 3% protein aqueous solution, which was sterilized with ultraviolet light for 15 min; the bacterial powder (self-produced strain BL99) was stirred and dispersed in cocoa butter (mass ratio 1:2), stirred for 2-4 min, and the oil and fat dispersed with bacterial powder and the protein aqueous solution were emulsified at a mass ratio of 1:2 at 12000 rpm for 4 min to obtain a core material emulsion; sodium alginate powder and konjac gum (1:10, w/w) were mixed and evenly dispersed in deionized water, and stirred at 400 rpm and 40°C for 30 min to obtain a mixed colloid solution with a colloidal raw material at a mass concentration of 0.5%; the inner core emulsion and the outer layer mixed colloid solution were magnetically stirred at 40°C and 1500 rpm for 20 s at a mass ratio of 1:6 to obtain a mixed solution, which was dropped into a 10% calcium chloride solution (pH 7) and reacted for 10 min to form microcapsules; the prepared microcapsules were placed in a 95°C water bath and heated for 1 min to cure into irreversible microcapsules, which were stored at 4°C.

The mixed colloid solution obtained in Example 13 was dripped into a 10% calcium chloride solution (pH 7) and reacted for 10 min to form microcapsules; the prepared microcapsules were placed in a 95°C water bath and heated for 1 min for curing, and the properties of the cured colloid were measured. After the measurement, the DSC endothermic peak of the colloid was at 198.62°C, the width of the endothermic peak was 62.82°C, the exothermic peak was at 280.71°C, and the turbidity at 25°C to 95°C was 1.47 cm$^{-1}$.

Example 14

Pea protein was weighed and dissolved in 100 mL of deionized water, the pH was adjusted to 7, and stirring was performed at 800 rpm for 3 h to prepare a 3% protein aqueous solution, which was sterilized with ultraviolet light for 15 min; the bacterial powder (self-produced strain BL99) was stirred and dispersed in cocoa butter (mass ratio 1:2), stirred for 2-4 min, and the oil and fat dispersed with bacterial powder and the protein aqueous solution were emulsified at a mass ratio of 1:2 at 12000 rpm for 4 min to obtain a core material emulsion; sodium alginate powder and gellan gum (1:10, w/w) were mixed and evenly dispersed in deionized water, and stirred at 400 rpm and 40°C for 30 min to obtain a mixed colloid solution with a colloidal raw material at a mass concentration of 0.5%; the core material emulsion and the outer layer mixed colloid solution were magnetically stirred at a mass ratio of 1:6 at 40°C and 1500 rpm for 20 s to obtain a mixed solution, which was dropped into a 10% calcium chloride solution (pH 7) and reacted for 10 min to form microcapsules; the prepared microcapsules were placed in a 95°C water bath and heated for 1 min to cure into irreversible microcapsules, which were stored at 4°C.

The mixed colloid solution of Example 14 was dropped into a 10% calcium chloride solution (pH 7), reacted for 10 min, and then placed in a 95°C water bath and heated for 1 min for curing. The properties of the cured colloid were measured. After the measurement, the DSC endothermic peak of the colloid was 192.65, the width of the endothermic peak was 57.13, the exothermic peak was 256.72, and the turbidity at 25°C to 95°C was 1.32 cm$^{-1}$.

Example 15

Casein was weighed and dissolved in 100 mL of deionized water, pH was adjusted to 7, stirring was performed at 800 rpm for 3 h, and a 3% protein aqueous solution was prepared and sterilized with ultraviolet light for 15 min; bacterial powder (self-produced strain BL99) was stirred and dispersed in cocoa butter (mass ratio 1:2), stirred for 2-4 min, and the oil and fat dispersed with bacterial powder and the protein aqueous solution were emulsified at a mass ratio of 1:2 at 12000 rpm for 4 min to obtain a core material emulsion; sodium alginate powder and curdlan gum (1:10, w/w) were mixed and evenly dispersed in deionized water, and stirred at 400 rpm and 40°C for 30 min to obtain a mixed colloid solution with a colloidal raw material at a mass concentration of 0.5%; the core material emulsion and the outer layer mixed colloid solution were magnetically stirred at a mass ratio of 1:6 at 40°C and 1500 rpm for 20 s to obtain a mixed solution, which was dropped into a 10% calcium chloride solution (pH 7) and reacted for 10 min to form microcapsules; the prepared microcapsules were placed in a 95°C water bath and heated for 1 min to cure into irreversible microcapsules, which were stored at 4°C.

The mixed colloid solution of Example 15 was dropped into a 10% calcium chloride solution (pH 7), reacted for 10 min, and then placed in a 95°C water bath and heated for 1 min for curing. The properties of the cured colloid were measured. The results were basically consistent with those of Example 9.

Example 16

Whey protein was weighed and dissolved in 100 mL of deionized water, pH was adjusted to 7, and stirring was performed at 800 rpm for 3 h to prepare a 3% protein aqueous solution, which was sterilized with ultraviolet light for 15 min; the bacterial powder (self-produced strain BL99) was stirred and dispersed in cocoa butter (mass ratio 1:2) and stirred for 2-4 min. The oil and fat dispersed with bacterial powder and protein aqueous solution were emulsified at a mass ratio of 1:2 at 12000 rpm for 4 min to obtain a core material emulsion; sodium alginate powder and curdlan gum (1:10, w/w) were mixed evenly and dispersed in deionized water, stirred at 400 rpm and 40°C for 30 min to obtain a mixed colloid solution with a colloidal raw material at a mass concentration of 0.5%; the core material emulsion and the outer layer mixed colloid solution were magnetically stirred at a mass ratio of 1:6 at 40°C and 1500 rpm for 20 s to obtain a mixed solution, which was dropped into a 10% calcium chloride solution (pH 7), and reacted for 10 min to form microcapsules; the prepared microcapsules were placed in a 95°C water bath and heated for 1 min to cure into irreversible microcapsules, which were stored at 4°C.

The mixed colloid solution of Example 16 was dropped into a 10% calcium chloride solution (pH 7), reacted for 10 min, and then placed in a 95°C water bath and heated for 1 min for curing. The properties of the cured colloid were measured, and the results were basically consistent with those of Example 9.

Example 17

Soy protein isolate was weighed and dissolved in 100 mL of deionized water, pH was adjusted to 7, and stirring was performed at 800 rpm for 3 h to prepare a 3% protein aqueous solution, which was sterilized with ultraviolet light for 15 min; the bacterial powder (self-produced strain BL99) was stirred and dispersed in cocoa butter (mass ratio 1:2) and stirred for 2-4 min, the oil and fat dispersed with bacterial powder and protein aqueous solution were emulsified at a mass ratio of 1:2 at 12000 rpm for 4 min to obtain a core material emulsion; sodium alginate powder and curdlan gum (1:10, w/w) were mixed evenly and dispersed in deionized water, and stirred at 400 rpm and 40°C for 30 min to obtain a mixed colloid solution with a colloidal raw material at a mass concentration of 0.5%; the core material emulsion and the outer layer mixed colloid solution were magnetically stirred at a mass ratio of 1:6 at 40°C and 1500 rpm for 20 s to obtain a mixed solution, which was dropped into a 10% calcium chloride solution (pH 7) , and reacted for 10 min to obtain microcapsules, and the prepared microcapsules were placed in a 95°C water bath and heated for 1 min to cure into irreversible microcapsules, which were then stored at 4°C.

The mixed colloid solution of Example 17 was dripped into a 10% calcium chloride solution (pH 7), reacted for 10 min, and then placed in a 95°C water bath and heated for 1 min for curing. The performance of the cured colloid was measured, and the results were basically consistent with those of Example 9.

The stability of the irreversible microcapsules obtained in Examples 1 to 17 and Comparative Examples 1 to 7 was tested, and the results are shown in Table 6.

High temperature shearing:

50 ml of sterile deionized water was placed in a beaker and kept warm in an 85°C water bath. After the deionized water in the beaker was completely heated to 85°C, 2.5 g of the sample was put into the beaker and stirred at 1800 rpm for 10 min using a stirrer, and the sample was taken for plate spreading.

75°C, 2 min: the sample was placed in sterile deionized water at 75°C for 2 min, and then taken out for plate spreading.

95°C, 1 min: the sample was placed in sterile deionized water at 95°C for 1 min, and then taken out for plate spreading.

Gastrointestinal digestion:

Simulated gastric juice-containing 8.5g/L NaCl, and 3g/L pepsin, at pH=1.8

Simulated intestinal juice-containing 8.5g/L NaCl, 3g/L bile salt, 10g/L pancreatic enzyme, and 10g/L trypsin, at pH=6.5

18ml of simulated gastric juice and simulated intestinal juice were placed in 50mL conical flasks respectively, and kept in a 37°C shaking incubator for 20 min. 2.0g of sample was weighed and placed in simulated gastric juice, and incubated at 37°C shaking incubator for 2h. The sample was filtered out and washed twice with sterile deionized water, and then placed in simulated intestinal juice, and incubated at 37°C shaking incubator for 4h. It was washed twice with sterile deionized water and plate spreading was performed.

Table 6 Measurement results of stability of irreversible microcapsule

| Sample | 75°C, 2min Δ bacteria count | 95°C, 1min Δ bacteria count | High temperature shearing Δ bacteria count | Gastrointestinal tract digestion Δ bacteria count |
|---|---|---|---|---|
| Example 1 | 2 | 2 | 1.1 | 0.9 |
| Example 2 | 1.9 | 1.8 | 1.1 | 1 |
| Example 3 | 1.7 | 1.7 | 1 | 0.7 |
| Example 4 | 1.6 | 1.5 | 1 | 0.6 |
| Example 5 | 2 | 2 | 1.1 | 0.9 |
| Example 6 | 2 | 2.1 | 1.1 | 0.8 |
| Example 7 | 1.6 | 1.7 | 0.9 | 0.6 |
| Example 8 | 1.7 | 1.6 | 0.8 | 0.6 |
| Example 9 | 2 | 1.9 | 1 | 1 |
| Example 10 | 2 | 2.1 | 1.2 | 1.2 |
| Example 11 | 1.8 | 1.8 | 1 | 0.8 |
| Example 12 | 1.7 | 1.7 | 0.7 | 0.7 |
| Example 13 | 1.6 | 1.6 | 0.9 | 0.7 |
| Example 14 | 1.6 | 1.5 | 0.7 | 0.6 |
| Example 15 | 1.2 | 1.3 | 0.7 | 0.6 |
| Example 16 | 1.3 | 1.5 | 0.8 | 0.5 |
| Example 17 | 1.4 | 1.5 | 0.8 | 0.6 |
| Comparative Example 1 | 2.6 | 2.7 | 1.4 | 1.3 |
| Comparative Example 2 | 2.8 | 2.9 | 1.6 | 1.5 |
| Comparative Example 3 | 2.4 | 2.5 | 1.5 | 1.4 |
| Comparative Example 4 | 2.5 | 2.3 | 1.6 | 1.7 |
| Comparative Example 5 | 2.6 | 2.7 | 1.7 | 1.5 |
| Comparative Example 6 | 2.5 | 2.6 | 1.2 | 1.6 |
| Comparative Example 7 | 2.5 | 2.3 | 1.3 | 1.5 |

Examples 18-21 and Comparative Examples 8-9

Casein was weighed and dissolved in 100 mL of deionized water, pH was adjusted to 7, and stirring was performed at 800 rpm for 3 h to prepare a 3% protein emulsion, which was sterilized with ultraviolet light for 15 min; bacterial powder (self-produced strain BL-99) was stirred and dispersed in the oil and fat composition (mass ratio 1:2), stirred for 2-4 min, and the oil and fat dispersed with probiotics and protein emulsion were emulsified at a mass ratio of 1:2 at 12000 rpm for 4 min to obtain a core material emulsion; sodium alginate powder and curdlan gum (1:10, w/w) were mixed and uniformly dispersed in deionized water, and stirred at 400 rpm and 40°C for 30 min to obtain a mixed colloid solution with a colloidal raw material at a mass concentration of 0.5%; the core material emulsion and the outer layer colloidal solution were magnetically stirred at a mass ratio of 1:6 at 40°C and 1500 rpm for 20 s to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C. See Table 7 for the process for oil and fat composition. The amount of catalyst used was 1% of the mass of the mixed oil. The performance of the oil and fat composition was measured and the results are shown in Table 8.

Table 7 Process for the oil and fat composition in Examples 18 to 21 and Comparative Examples 8 to 9

| Number | raw materials of the oil and fat composition | ratio | saturated fatty acids: unsaturated fatty acids | catalyst | reaction temperature | reaction time |
|---|---|---|---|---|---|---|
| Example 18 | Palm kernel oil: coconut oil: palm stearin | 10:50:40 | 70:30 | Lipase | 35 °C | 2h |
| Example 19 | Rapeseed oil: rice bran oil: butter | 10:10:80 | 40:60 | Lipase | 35 °C | 4h |

(continued)

| Number | raw materials of the oil and fat composition | ratio | saturated fatty acids: unsaturated fatty acids | catalyst | reaction temperature | reaction time |
|---|---|---|---|---|---|---|
| Example 20 | Soybean oil: sunflower oil: coconut oil | 20:50:30 | 25:75 | Sodium methoxide | 120 °C | 45 min |
| Example 21 | Rapeseed oil: corn oil: cocoa butter | 10:10:80 | 50:50 | Sodium methoxide | 130 °C | 30 min |
| Comparative Example 8 | Cocoa butter: coconut oil: palm stearin | 20:20:60 | 80:20 | Lipase | 30 °C | 1 h |
| Comparative Example 9 | Rice bran oil: corn oil: soybean oil | 30:30:40 | 20:80 | Sodium methoxide | 140 °C | 20 min |

Table 8 Performance test results of the oil and fat compositions in Examples 18 to 21 and Comparative Examples 8 to 9

| Number | DSC endothermic peak of the oil and fat | DSC exothermic peak of the oil and fat | Curve slope of solid fat | Viscosity mPa·s |
|---|---|---|---|---|
| Example 18 | 40.78 | 222.48 | -2.86 | 25.45 |
| Example 19 | 34.75 | 230.10 | -1.22 | 60.42 |
| Example 20 | 45.72 | 230.57 | -2.45 | 28.40 |
| Example 21 | 47.01 | 240.36 | -1.76 | 80.45 |
| Comparative Example 8 | 25.95 | 210.19 | -3.51 | 15.15 |
| Comparative Example 9 | 52.09 | 262.24 | -0.56 | 91.50 |

Examples 22-25 and Comparative Examples 10-11

Casein was weighed and dissolved in 100 mL of deionized water, pH was adjusted to 7, and stirring was performed at 800 rpm for 3 h to prepare a 3% protein emulsion, which was sterilized with ultraviolet light for 15 min; the bacterial powder (self-produced strain BL-99) was stirred and dispersed in the mixed oil and fat (mass ratio 1:2), stirred for 2-4 min, and the oil and fat with dispersed probiotics and the protein emulsion were emulsified at a mass ratio of 1:2 at 12000 rpm for 4 min to obtain a core material emulsion; sodium alginate powder and curdlan gum (1:10, w/w) were mixed and evenly dispersed in deionized water, and stirred at 400 rpm and 40°C for 30 min to obtain a mixed colloidal solution with a colloidal raw material at a mass concentration of 0.5%; the core material emulsion and the outer layer colloidal solution were magnetically stirred at a mass ratio of 1:6 at 40°C and 1500 rpm for 20 s to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C. The composition of the oil and fat composition is shown in Table 9, and the performance of the oil and fat composition was measured, and the results are shown in Table 10.

Table 9 process of the oil and fat composition in Examples 22 to 25 and Comparative Examples 10 to 11

| Number | Mixed oil and fat | Ratio |
|---|---|---|
| Example 22 | Rice bran oil: Coconut oil | 30:70 |
| Example 23 | Corn oil: Palm stearin | 20:80 |
| Example 24 | Rapeseed oil: Sunflower oil: Butter | 10:20:70 |
| Example 25 | Soybean oil: Corn oil: Cocoa butter | 10:10:80 |
| Comparative Example 10 | Palm stearin: Coconut oil | 80:20 |
| Comparative Example 11 | Soybean oil: Corn oil | 60:40 |

Table 10 Measurement results of performance of the oil and fat compositions in Examples 22 to 25 and Comparative Examples 10 to 11

| Number | DSC endothermic peak of the oil and fat | DSC exothermic peak of the oil and fat | Curve slope of solid fat | Viscosity mPa·s |
|---|---|---|---|---|
| Example 22 | 47.82 | 242.21 | -2.61 | 26.50 |
| Example 23 | 32.58 | 245.10 | -1.60 | 58.45 |
| Example 24 | 42.27 | 225.51 | -2.22 | 38.40 |
| Example 25 | 41.18 | 230.68 | -1.87 | 69.30 |
| Comparative Example 10 | 24.56 | 208.97 | -3.11 | 10.50 |
| Comparative Example 11 | 54.93 | 255.48 | -0.65 | 92.55 |

Examples 26-29 and Comparative Examples 12-13

Casein was weighed and dissolved in 100 mL of deionized water, pH was adjusted to 7, and stirring was performed at 800 rpm for 3 h to prepare a 3% protein emulsion, which was sterilized with ultraviolet light for 15 min; bacterial powder (self-produced strain BL-99) was stirred and dispersed in the fractionated oil (mass ratio 1:2), and stirred for 2-4 min. The oil and fat dispersed with probiotics and the protein emulsion were emulsified at a mass ratio of 1:2 at 12000 rpm for 4 min to obtain a core material emulsion; sodium alginate powder and curdlan gum (1:10, w/w) were mixed and evenly dispersed in deionized water, and stirred at 400 rpm and 40°C for 30 min to obtain a mixed colloidal solution with a colloidal raw material at a mass concentration of 0.5%; the core material emulsion and the outer layer colloidal solution were magnetically stirred at a mass ratio of 1:6 at 40°C and 1500 rpm for 20-30 s to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C. The oil and fat composition was a fractionated oil, the types of which are shown in Table 11, and the properties of the oil and fat composition were measured, and the results are shown in Table 12.

Table 11 The oil and fat composition in Examples 26 to 29 and Comparative Examples 12 to 13

| Number | Fractionated oil |
|---|---|
| Example 26 | 44° palm oil |
| Example 27 | 33° palm oil |
| Example 28 | 24° palm oil |
| Example 29 | 14° palm oil |
| Comparative Example 12 | palm stearin |
| Comparative Example 13 | 8° palm oil |

Table 12 Measurement results of performance of the oil and fat compositions in Examples 26 to 29 and Comparative Examples 12 to 13

| Number | DSC endothermic peak of the oil and fat | DSC exothermic peak of the oil and fat | Curve slope of solid fat | Viscosity mPa·s |
|---|---|---|---|---|
| Example 26 | 45.12 | 246.12 | -2.82 | 25.65 |
| Example 27 | 34.75 | 235.81 | -1.18 | 86.25 |
| Example 28 | 43.02 | 245.28 | -2.66 | 39.30 |
| Example 29 | 38.10 | 232.09 | -1.73 | 49.40 |
| Comparative Example 12 | 22.55 | 212.75 | -3.50 | 15.05 |
| Comparative Example 13 | 58.93 | 265.67 | -0.88 | 90.15 |

Examples 30-33 and Comparative Examples 14-15

Casein was weighed and dissolved in 100 mL of deionized water, pH was adjusted to 7, and stirring was performed at 800 rpm for 3 h to prepare a 3% protein emulsion, which was sterilized with ultraviolet light for 15 min; bacterial powder (self-produced strain BL-99) was stirred and dispersed in hydrogenated vegetable oil (mass ratio 1:2), and stirred for 2-4 min. The oil and fat dispersed with probiotics and the protein emulsion were emulsified at a mass ratio of 1:2 at 12000 rpm for 4 min to obtain a core material emulsion. The hydrogenation process is shown in Table 13. Curdlan gum was mixed with sodium alginate powder and evenly dispersed in deionized water (curdlan gum: sodium alginate = 10:1), stirred at 400 rpm and 40°C for 30 min to obtain a mixed colloid solution with a colloid concentration of 0.5%; the core material emulsion and the outer layer mixed colloid solution were stirred at a mass ratio of 1:6 at 40°C and 1500 rpm for 20 s to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C (the mixed colloid solution was dropped into a 10% calcium chloride solution (pH 7), reacted for 10 min to form microcapsules; the prepared microcapsules were placed in a 70°C water bath and heated for 1 min for curing, and the properties of the cured colloid were tested. The DSC curve of the wall material formed after the colloid was cured in this exampled had an endothermic peak at 198.92°C and an exothermic peak at 277.15°C; the turbidity of the colloid at 25°C to 95°C was 1.42 cm$^{-1}$). The properties of hydrogenated vegetable oil were measured, and the results are shown in Table 14.

Table 13 Hydrogenation process in Examples 30 to 33 and Comparative Examples 14 to 15

| Number | Oil and fat as raw material | Reaction pressure | Reaction temperature | Reaction time |
|---|---|---|---|---|
| Example 30 | Soybean oil | 0.02 MPa | 100 °C | 2 h |
| Example 31 | Peanut oil | 0.5 MPa | 180 °C | 4 h |
| Example 32 | Flaxseed oil | 0.1 MPa | 130 °C | 3 h |
| Example 33 | Rapeseed oil | 0.3 MPa | 150 °C | 3 h |
| Comparative Example 14 | Soybean oil | 1 MPa | 200 °C | 5 h |
| Comparative Example 15 | Flaxseed oil | 0.01 MPa | 80 °C | 1 h |

Table 14 Performance test results of hydrogenated vegetable oil in Examples 30-33 and Comparative Examples 14-15

| Number | DSC endothermic peak of the oil and fat | DSC exothermic peak of the oil and fat | Curve slope of solid fat | Viscosity mPa·s |
|---|---|---|---|---|
| Example 30 | 47.58 | 236.81 | -2.26 | 26.25 |
| Example 31 | 32.75 | 225.28 | -1.62 | 82.50 |
| Example 32 | 40.23 | 245.19 | -2.48 | 29.30 |
| Example 33 | 38.01 | 232.90 | -1.59 | 59.45 |
| Comparative Example 14 | 22.95 | 202.50 | -3.58 | 16.05 |
| Comparative Example 15 | 55.19 | 259.67 | -0.81 | 98.15 |

The stability of the microcapsules obtained in Examples 18 to 33 and Comparative Examples 8 to 15 was tested, and the results are shown in Table 15.

High temperature shearing:

50 ml of sterile deionized water was put in a beaker and placed in an 85°C water bath to keep warm. After the deionized water in the beaker was completely heated to 85°C, 2.5 g of the sample was put into the beaker, stirred at 1800 rpm for 10 min using a stirrer, and the sample was taken for plate spreading.

75°C, 2 min: the sample was placed in sterile deionized water at 75°C for 2 min, and taken out for plate spreading.

95°C, 1 min: the sample was placed in sterile deionized water at 95°C for 1 min, and taken out for plate spreading.

Gastrointestinal digestion:

Simulated gastric juice-containing 8.5g/L NaCl, 3g/L pepsin, pH=1.8

Simulated intestinal juice-containing 8.5g/L NaCl, 3g/L bile salt, 10g/L pancreatic enzyme, 10g/L trypsin, pH=6.5

18ml of simulated gastric juice and simulated intestinal juice were placed in 50mL conical flasks respectively, and kept in a 37°C shaking incubator for 20min. 2.0g of sample was weighed and placed in simulated gastric juice, and incubated at 37°C shaking incubator for 2h. The sample was filtered out and washed twice with sterile deionized water, then placed in simulated intestinal juice, incubated at 37°C shaking incubator for 4h, washed twice with sterile deionized water, and subjected to plate spreading.

Stability at room temperature storage:
The microcapsule samples were placed at room temperature for 28 days and then subjected to plate spreading.

Table 15 Heat resistance and shear resistance parameters of microcapsules of Examples 18 to 33 and Comparative Examples 8 to 15

| Sample | 75 °C, 2 min Δ Bacteria count | 95 °C, 1 min Δ Bacteria count | Shear resistance Δ Bacteria count | Gastric acid resistance Δ Bacteria count | Storage at room temperature Δ Bacteria count |
|---|---|---|---|---|---|
| Example 18 | 1.9 | 1.8 | 1.3 | 0.8 | - |
| Example 19 | 1.8 | 1.9 | 1.3 | 0.8 | - |
| Example 20 | 1.7 | 1.6 | 1.1 | 0.7 | 0.6 |
| Example 21 | 1.5 | 1.5 | 1.1 | 0.6 | 0.5 |
| Example 22 | 1.9 | 1.8 | 1.2 | 0.8 | - |
| Example 23 | 1.8 | 1.8 | 1.3 | 0.9 | - |
| Example 24 | 1.6 | 1.7 | 1 | 0.7 | 0.6 |
| Example 25 | 1.6 | 1.7 | 1 | 0.6 | 0.5 |
| Example 26 | 2 | 2.1 | 1.3 | 1.1 | - |
| Example 27 | 2 | 1.8 | 1.3 | 1.2 | - |
| Example 28 | 1.8 | 1.8 | 1.2 | 0.8 | 0.5 |
| Example 29 | 1.6 | 1.7 | 0.9 | 0.7 | 0.6 |
| Example 30 | 1.6 | 1.6 | 0.7 | 0.8 | - |
| Example 31 | 1.5 | 1.5 | 0.7 | 0.7 | - |
| Example 32 | 1.4 | 1.5 | 0.8 | 0.6 | 0.5 |
| Example 33 | 1.4 | 1.4 | 0.7 | 0.6 | 0.5 |
| Comparative Example 8 | 2.4 | 2.5 | 1.6 | 1.2 | - |
| Comparative Example 9 | 2.6 | 2.7 | 1.5 | 1.4 | - |
| Comparative Example 10 | 2.4 | 2.4 | 1.6 | 1.4 | - |
| Comparative Example 11 | 2.4 | 2.3 | 1.5 | 1.6 | - |
| Comparative Example 12 | 2.4 | 2.5 | 1.6 | 1.4 | - |
| Comparative Example 13 | 2.5 | 2.6 | 1.6 | 1.6 | - |
| Comparative Example 14 | 2.6 | 2.5 | 1.2 | 1.5 | - |
| Comparative Example 15 | 2.7 | 2.5 | 1.2 | 1.2 | - |

Example 34

Soy protein isolate was weighed and dissolved in 100 mL of deionized water, pH was adjusted to 7, and stirring was performed at 800 rpm for 3 h to prepare a 3% protein emulsion, which was sterilized with ultraviolet light for 15 min; vitamin C was stirred and dispersed in the oil and fat composition of Example A3 described above (mass ratio 1:2), and stirred for 2-4 min. The oil and fat dispersed with vitamin C and the protein emulsion were emulsified at a mass ratio of 1:2 at 12000 rpm for 4 min to obtain a core material emulsion; curdlan gum and sodium alginate powder were mixed respectively and evenly dispersed in deionized water (curdlan gum: sodium alginate = 10:1), and stirred at 400 rpm and 40°C for 30 min to obtain a mixed colloid solution with a latex concentration of 0.5%; the core material emulsion and the outer layer mixed colloid solution were magnetically stirred at a mass ratio of 1:6 at 40°C and 1500 rpm for 20 s to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C.

The mixed colloid solution was dropped into a 10% calcium chloride solution (pH 7) and reacted for 10 min to form microcapsules; the prepared microcapsules were placed in a 70°C water bath and heated for 1 min for curing, and the performance of the cured colloid was tested, which was basically consistent with Examples 30 to 33 of the present disclosure.

Example 35

Soy protein isolate was weighed and dissolved in 100 mL of deionized water, pH was adjusted to 7, and stirring was performed at 800 rpm for 3 h to prepare a 3% protein emulsion, which was sterilized with ultraviolet light for 15 min;

lactoferrin was stirred and dispersed in the oil and fat composition of Example A7 described above (mass ratio 1:2), and stirred for 2-4 min. The oil and fat dispersed with lactoferrin and the protein emulsion were emulsified at a mass ratio of 1:2 at 12000 rpm for 4 min to obtain a core material emulsion; curdlan gum and sodium alginate powder were mixed respectively and evenly dispersed in deionized water (curdlan gum: sodium alginate = 10:1), and stirred at 400 rpm and 40°C for 30 min to obtain a mixed colloid solution with a colloid mass concentration of 0.5%; the core material emulsion and the outer layer mixed colloid solution were magnetically stirred at a mass ratio of 1:6 at 40°C and 1500 rpm for 20-30 s to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C.

The mixed colloid solution was dropped into a 10% calcium chloride solution (pH 7) and reacted for 10 min to form microcapsules; the prepared microcapsules were placed in a 70°C water bath and heated for 1 min for curing, and the performance of the cured colloid was tested, which was basically consistent with Examples 30 to 33 of the present disclosure.

Example 36

Soy protein isolate was weighed and dissolved in 100 mL of deionized water, pH was adjusted to 7, and stirring was performed at 800 rpm for 3 h to prepare a 3% protein emulsion, which was sterilized with ultraviolet light for 15 min; anthocyanidin was stirred and dispersed in the oil and fat composition of Example A10 described above (mass ratio 1:2), and stirred for 2-4 min, the oil and fat dispersed with anthocyanidin and the protein emulsion were emulsified at a mass ratio of 1:2 at 12000 rpm for 4 min to obtain a core material emulsion; curdlan gum and sodium alginate powder were mixed and dispersed respectively in deionized water (curdlan gum: sodium alginate = 10:1), and stirred at 400 rpm and 40°C for 30 min to obtain a mixed colloid solution with a colloidal mass concentration of 0.5%; the core material emulsion and the outer layer mixed colloid solution were magnetically stirred at a mass ratio of 1:6 at 40°C and 1500 rpm for 20-30 s to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C.

The mixed colloid solution was dropped into a 10% calcium chloride solution (pH 7) and reacted for 10 min to form microcapsules; the prepared microcapsules were placed in a 70°C water bath and heated for 1 min for curing, and the performance of the cure colloid was tested. The test results were basically consistent with those of Examples 30 to 33.

The performance of the microcapsules obtained in Examples 34 to 36 was tested, and the results are shown in Table 16.

Table 16 Performance test results of the microcapsules of Examples 34 to 36

| Sample | Encapsulation rate | pH 2, 4h stability | pH 7, 4h stability | Gastric release rate (2h) | Intestinal release rate (4h) |
|---|---|---|---|---|---|
| Example 34 | 82.6% | 94.0% | 63.5% | 36.04% | 71.6% |
| Example 35 | 80.1% | 93.5% | 68.5% | 46.04% | 75.6% |
| Example 36 | 74.3% | 98.2% | 77.8% | 43.1% | 82.0% |

Example 37

Casein was weighed and dissolved in 100 mL of deionized water, pH was adjusted to 7, and stirring was performed at 800 rpm for 3 h to prepare a 3% protein emulsion, which was sterilized with ultraviolet light for 15 min; bacterial powder (self-produced strain BL-99) was stirred and dispersed in the oil and fat composition of Example A3 described above (mass ratio 1:2), and stirred for 2-4 min, and the oil and fat dispersed with probiotics and the protein emulsion were emulsified at 12000 rpm for 4 min at a mass ratio of 1:2 to obtain a core material emulsion; curdlan gum and sodium alginate powder were mixed and evenly dispersed respectively in deionized water (curdlan gum: sodium alginate = 10:1), and stirred at 400 rpm and 40°C for 30 min to obtain a mixed colloid solution with a colloid mass concentration of 0.5%; the core material emulsion and the outer layer mixed colloid solution were magnetically stirred at a mass ratio of 1:6 at 40°C and 1500 rpm for 20 s to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C.

The mixed colloid solution was dropped into a 10% calcium chloride solution (pH 7) and reacted for 10 min to form microcapsules; the prepared microcapsules were placed in a 70°C water bath and heated for 1 for curing, and the performance of the cure colloid was tested, which was basically consistent with Examples 30 to 33 of the present disclosure.

Example 38

Casein was weighed and dissolved in 100 mL of deionized water, pH was adjusted to 7, and stirring was performed at 800 rpm for 3 h to prepare a 3% protein emulsion, which was sterilized with ultraviolet light for 15 min; the bacterial powder (self-produced strain BL-99) was stirred and dispersed in the oil and fat composition of Example A6 described above (mass ratio 1:2), and stirred for 2-4 min, and the oil and fat dispersed with probiotics and the protein emulsion were emulsified at a mass ratio of 1:2 at 12000 rpm for 4 min to obtain a probiotic/oil and fat-protein emulsion; curdlan gum was mixed with sodium alginate powder and evenly dispersed in deionized water (curdlan gum: sodium alginate = 10:1), and stirred at 400 rpm and 40°C for 30 min to obtain a mixed colloid solution with a colloid mass concentration of 0.5%; the core material emulsion and the outer layer mixed colloid solution were magnetically stirred at a mass ratio of 1:6 at 40°C and 1500 rpm for 20-30 s to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C.

The mixed colloid solution was dropped into a 10% calcium chloride solution (pH 7) and reacted for 10 min to form microcapsules; the prepared microcapsules were placed in a 70°C water bath and heated for 1 min for curing, and the performance of the cured colloid was tested, which was basically consistent with Examples 30 to 33 of the present disclosure.

Example 39

Casein was weighed and dissolved in 100 mL of deionized water, pH was adjusted to 7, and stirring was performed at 800 rpm for 3 h to prepare a 3% protein emulsion, which was sterilized with ultraviolet light for 15 min; bacterial powder (self-produced strain BL-99) was stirred and dispersed in the oil and fat composition of Example 10 described above (mass ratio 1:2), and stirred for 2-4 min, and the oil and fat dispersed with probiotics and the protein emulsion were emulsified at a mass ratio of 1:2 at 12000 rpm for 4 min to obtain a core material emulsion; curdlan gum and sodium alginate powder were mixed and evenly dispersed in deionized water (curdlan gum: sodium alginate = 10:1), and stirred at 400 rpm and 40°C for 30 min to obtain a mixed colloid solution with a colloid mass concentration of 0.5%; the core material emulsion and the outer layer mixed colloid solution were magnetically stirred at 40°C and 1500 rpm for 20 s at a mass ratio of 1:6 to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C.

The mixed colloid solution was dropped into a 10% calcium chloride solution (pH 7) and reacted for 10 min to form microcapsules; the prepared microcapsules were placed in a 70°C water bath and heated for 1 min for curing, and the performance of the cured colloid was tested, which was basically consistent with Examples 30 to 33 of the present disclosure.

Example 40

Casein was weighed and dissolved in 100 mL of deionized water, pH was adjusted to 7, and stirring was performed at 800 rpm for 3 h to prepare a 3% protein emulsion, which was sterilized with ultraviolet light for 15 min; the bacterial powder (self-produced strain BL-99) was stirred and dispersed in the oil and fat composition of Example 10 described above (mass ratio 1:2), and stirred for 2-4 min, and the oil and fat dispersed with probiotics and protein emulsion were emulsified at a mass ratio of 1:2 at 12000 rpm for 4 min to obtain a core material emulsion; curdlan gum was mixed with sodium alginate powder and evenly dispersed in deionized water (curdlan gum: sodium alginate = 10:1), and stirred at 400 rpm and 40°C for 30 min to obtain a mixed colloid solution with a colloid mass concentration of 0.5%; the core material emulsion and the outer layer mixed colloid solution were magnetically stirred at a mass ratio of 1:6 at 40°C and 1500 rpm for 20 s to obtain a mixed solution, which was dropped into a 10% calcium chloride solution (pH 7) to form microcapsules; the prepared microcapsules were placed in a 95°C water bath and heated for curing to form irreversible microcapsules, which were stored at 4°C.

The mixed colloid solution was dropped into a 10% calcium chloride solution (pH 7) and reacted for 10 min to form microcapsules; the prepared microcapsules were placed in a 70°C water bath and heated for 1 min for curing, and the performance of the cured colloid was tested, which was basically consistent with that of Examples 30 to 33.

The performance of the microcapsules obtained in Examples 37 to 40 was tested, and the results are shown in Table 17.

Table 17 Performance test results of microcapsules of Examples 37 to 40

| Sample | Encapsulation rate | Gastric release rate (2h) | Intestinal release rate (4h) | Lipid sphere particle size (μm) |
|---|---|---|---|---|
| Example 37 | 82.6% | 36.04% | 71.6% | 200 |
| Example 38 | 74.3% | 43.1% | 82.0% | 500 |
| Example 39 | 71.1% | 40.5% | 80.5% | 1000 |
| Example 40 | 74.8% | 39.1% | 78.1% | 2000 |

Examples 41-44 and Comparative Examples 16-17

Pea protein was weighed and dissolved in 100 mL of deionized water, pH was adjusted to 7, and stirring was performed at 800 rpm for 3 h to prepare a 3% protein emulsion, which was sterilized with ultraviolet light for 15 min; the bacterial powder was stirred and dispersed in shortening (palm oil source, having DSC endothermic peak at around 48°C and DSC exothermic peak at around 241°C, with a melting curve slope of -1.85, and a viscosity of 25.2 mPa·s) at a mass ratio of 1:2, and stirred for 2-4 min, and the oil and fat dispersed with bacterial powder and protein emulsion were emulsified at a mass ratio of 1:2 at 12000 rpm for 4 min to obtain a core material emulsion; sodium alginate powder and curdlan gum (1:10, w/w) were mixed and evenly dispersed in deionized water to obtain an outer layer colloid solution, and the specific preparation parameters are shown in Table 18; the core material emulsion and the outer layer colloid solution were magnetically stirred at a mass ratio of 1:6 at 40°C and 1500 rpm for 20 s to obtain a mixed solution, which was then dropped into a 10% calcium chloride solution (pH 7) and reacted for 10 min to form microcapsules; the prepared microcapsules were placed in a 95°C water bath and heated for 1 min to cure into irreversible microcapsules, which were stored at 4°C.

Table 18 Preparation parameters of Examples 41-44 and Comparative Examples 16-17

| Number | Colloid concentration (%) | Stirring temperature (°C) | Stirring speed (rpm) |
|---|---|---|---|
| Example 41 | 1 | 50 | 1000 |
| Example 42 | 0.1 | 30 | 200 |
| Example 43 | 0.3 | 40 | 400 |
| Example 44 | 0.5 | 45 | 800 |
| Comparative Example 16 | 1.5 | 55 | 1200 |
| Comparative Example 17 | 0.05 | 20 | 100 |

The outer layer colloid solution in Examples 41-44 and Comparative Examples 16-17 was dropped into a 10% calcium chloride solution (pH 7), reacted for 10 min, and then placed in a 95°C water bath and heated for 1 min for curing. The properties of the cured colloid were measured, and the results are shown in Table 19.

Table 19 Performance test results of the cured colloid in Examples 41-44 and Comparative Examples 16-17

| Number | DSC endothermic peak of colloid | DSC exothermic peak of colloid | Turbidity at 25°C to 95°C |
|---|---|---|---|
| Example 41 | 202.48 | 280.88 | 1.45 |
| Example 42 | 190.10 | 269.24 | 1.40 |
| Example 43 | 192.57 | 271.76 | 1.41 |
| Example 44 | 198.36 | 278.40 | 1.42 |
| Comparative Example 16 | 218.19 | 290.99 | 1.50 |
| Comparative Example 17 | 182.24 | 240.27 | 1.35 |

Examples 45-48 and Comparative Examples 18-19

Pea protein was weighed and dissolved in 100 mL of deionized water, pH was adjusted to 7, and stirring was performed at 800 rpm for 3 h to prepare a 3% protein emulsion, which was sterilized with ultraviolet light for 15 min; the bacterial powder was stirred and dispersed in shortening (palm oil source, having DSC endothermic peak at around 48°C and DSC exothermic peak at around 241°C, with a melting curve slope of -1.85 and a viscosity of 25.2 mPa·s) at a mass ratio of 1:2, and stirred for 2-4 min, and the oil and fat dispersed with bacterial powder and the protein emulsion were emulsified at a mass ratio of 1:2 at 12000 rpm for 4 min to obtain a core material emulsion; sodium alginate powder and curdlan gum (1:10, w/w) were mixed and evenly dispersed in deionized water, and stirred at 400 rpm and 40°C for 30 min to obtain an outer layer colloid solution with a colloidal mass concentration of 0.5%; the core material emulsion and the outer layer colloid solution were magnetically stirred at 40°C and 1500 rpm for 20 s to obtain a mixed solution, which was dropped into a 10% calcium chloride solution (pH7) and reacted for 10 min to form microcapsules. The specific parameters are shown in Table 20; the prepared microcapsules were placed in a 95°C water bath and heated for 1 min to cure into irreversible microcapsules, which were stored at 4°C.

Table 20 Preparation parameters of Examples 45-48 and Comparative Examples 18-19

| Number | Core material emulsion: outer layer colloid solution | Stirring temperature (°C) | Stirring speed (rpm) |
|---|---|---|---|
| Example 45 | 1:2 | 30 | 500 |
| Example 46 | 1:7 | 50 | 2000 |
| Example 47 | 1:3 | 40 | 1500 |
| Example 48 | 1:5 | 45 | 800 |
| Comparative Example 18 | 1:10 | 55 | 1200 |
| Comparative Example 19 | 1:1 | 20 | 200 |

The outer layer colloid solution in Examples 45-48 and Comparative Examples 18-19 was dropped into a 10% calcium chloride solution (pH 7), reacted for 10 min, and then placed in a 95°C water bath and heated for 1 min for curing, and the properties of the cured colloid were measured. The results were basically consistent with those of Example 44.

Examples 49-52 and Comparative Examples 20-21

Pea protein was weighed and dissolved in 100 mL of deionized water, pH was adjusted to 7, and stirring was performed at 800 rpm for 3 h to prepare a 3% protein emulsion, which was sterilized with ultraviolet light for 15 min; the bacterial powder was stirred and dispersed in shortening (palm oil source, having DSC endothermic peak at around 48°C and DSC exothermic peak at around 241°C, with a melting curve slope of -1.85 and a viscosity of 25.2 mPa·s) at a mass ratio of 1:2, and stirred for 2-4 min, and the oil and fat dispersed with bacterial powder and protein emulsion were emulsified at a mass ratio of 1:2 at 12000 rpm for 4 min to obtain a core material emulsion; sodium alginate powder and curdlan gum (1:10, w/w) were mixed and evenly dispersed in deionized water, and stirred at 400 rpm and 40°C for 30min to obtain an outer layer colloid solution with a colloid mass concentration of 0.5%; the inner core latex and the outer layer colloid solution (1:6) were magnetically stirred at 40°C and 1500 rpm for 20s to obtain a mixed solution, which was dropped into a 10% calcium chloride solution (pH 7) and reacted for 10 min to form microcapsules; the prepared microcapsules were placed in a water bath and heated for 1 min to cure into irreversible microcapsules, which were stored at 4°C. The water bath temperature is shown in Table 21.

The outer layer colloid solution in Examples 49 to 52 and Comparative Examples 20 to 21 was dropped into a 10% calcium chloride solution (pH 7), reacted for 10 min, and then placed in a water bath at 70°C to 95°C and heated for 1 min for curing. The properties of the cured colloid were measured, and the results are shown in Table 21.

Table 21 Preparation parameters of Examples 49 to 52 and Comparative Examples 20 to 21 and properties of the cured colloid

| Number | Curing temperature (°C) | DSC endothermic peak of colloid | DSC exothermic peak of colloid | Turbidity at 25°C to 95°C |
|---|---|---|---|---|
| Example 49 | 95 | 198.92 | 277.15 | 1.42 |
| Example 50 | 75 | 191.10 | 272.40 | 1.41 |
| Example 51 | 80 | 193.84 | 278.83 | 1.42 |
| Example 52 | 85 | 193.36 | 274.72 | 1.45 |
| Comparative Example 20 | 70 | 186.19 | 237.92 | 1.32 |
| Comparative Example 21 | 100 | 201.24 | 284.37 | 1.33 |

Comparative Example 22

Pea protein was weighed and dissolved in 100 mL of deionized water, pH was adjusted to 7, and stirring was performed at 800 rpm for 3 h, and a 3% protein emulsion was prepared and sterilized with ultraviolet light for 15 min; the bacterial powder was stirred and dispersed in shortening (palm oil source, having DSC endothermic peak at around 48°C and DSC exothermic peak at around 241°C, with a melting curve slope of -1.85 and a viscosity of 25.2 mPa·s) at a mass ratio of 1:2, and stirred for 2-4 min, and the oil and fat dispersed with bacterial powder and the protein emulsion were emulsified at a mass ratio of 1:2 at 12000 rpm for 4 min to obtain a core material emulsion; sodium alginate powder and gellan gum (1:10, w/w) were mixed and evenly dispersed in deionized water, and stirred at 400 rpm and 40°C for 30 min to obtain an outer layer colloid solution with a colloid mass concentration of 0.5%; the core material emulsion and the outer layer colloid solution were magnetically stirred at a mass ratio of 1:6 at 40°C and 1500 rpm for 20 s to obtain a mixed solution, which was dropped into a 10% calcium chloride solution (pH 7) and reacted for 10 min to form microcapsules; the prepared microcapsules were placed in a 70°C water bath and heated for 1 min to cure into irreversible microcapsules, which were stored at 4°C.

The mixed colloid solution in comparative example 22 was dropped into a 10% calcium chloride solution (pH 7) and reacted for 10 min to form microcapsules; the prepared microcapsules were placed in a 70°C water bath and heated for 1 min for cuing, and the performance of the cured colloid was tested. After testing, the DSC endothermic peak of the colloid was at 182.65°C, the width of the endothermic peak was 54.72, the exothermic peak was at 250.30°C, and the turbidity at 25°C to 95°C was 1.36 cm$^{-1}$.

Example 53

Pea protein was weighed and dissolved in 100 mL of deionized water, pH was adjusted to 7, and stirring was performed at 800 rpm for 3 h to prepare a 3% protein emulsion and sterilized with ultraviolet light for 15 min; the bacterial powder was stirred and dispersed in shortening (palm oil source, having DSC endothermic peak at around 48°C and DSC exothermic peak at around 241°C, with a melting curve slope of -1.85 and a viscosity of 25.2 mPa·s) at a mass ratio of 1:2, and stirred for 2-4 min, and the oil and fat dispersed with bacterial powder and the protein emulsion were emulsified at a mass ratio of 1:2 at 12000 rpm for 4 min to obtain a core material emulsion; sodium alginate powder was mixed with konjac gum (1:10, w/w) and evenly dispersed in deionized water, and stirred at 40°C and 400 rpm for 30 min to obtain an outer layer colloid solution with a colloid mass concentration of 0.5%; the core material emulsion and the outer layer colloid solution were magnetically stirred at a mass ratio of 1:6 at 40°C and 1500 rpm for 20 s to obtain a mixed solution, which was dropped into a 10% calcium chloride solution (pH 7) and reacted for 10 min to form microcapsules; the prepared microcapsules were placed in a 95°C water bath and heated for 1 min to cure into irreversible microcapsules, which were stored at 4°C.

The outer layer colloid solution in Example 53 was dropped into a 10% calcium chloride solution (pH 7) and reacted for 10 min to form microcapsules; the prepared microcapsules were placed in a 95°C water bath and heated for 1 min for curing, and the cured colloid was tested. After test, the DSC endothermic peak of the colloid was at 198.62°C, the width of the endothermic peak was 62.82, the exothermic peak was at 280.71°C, and the turbidity at 25°C to 95°C was 1.47 cm$^{-1}$.

Example 54

Pea protein was weighed and dissolved in 100 mL of deionized water, pH was adjusted to 7, and stirring was performed at 800 rpm for 3 h to prepare a 3% protein emulsion, which was sterilized with ultraviolet light for 15 min; the bacterial powder was stirred and dispersed in shortening (cocoa butter source, having DSC endothermic peak at around 30°C and DSC exothermic peak at around 200°C, with a melting curve slope of -2.45 and a viscosity of 28.0 mPa·s) at a mass ratio of 1:2, and stirred for 2-4 min, and the oil and fat dispersed with bacterial powder and protein emulsion were emulsified at a mass ratio of 1:2 at 12000 rpm for 4 min to obtain a core material emulsion; sodium alginate powder and gellan gum (1:10, w/w) were mixed and evenly dispersed in deionized water, and stirred at 400 rpm and 40°C for 30 min to obtain an outer layer colloid solution with a colloidal mass concentration of 0.5%; the core material emulsion and the outer layer colloid solution (1:6) were magnetically stirred at 40°C and 1500 rpm for 20 s to obtain a mixed solution, which was then dropped into a 10% calcium chloride solution (pH 7) and reacted for 10 min to form microcapsules; the prepared microcapsules were placed in a 95°C water bath and heated for 1 min to cure into irreversible microcapsules, which were stored at 4°C.

The outer layer colloid solution of Example 54 was dropped into a 10% calcium chloride solution (pH 7), reacted for 10 min, and then placed in a 95°C water bath and heated for 1 min for curing, and the properties of the cured colloid were measured. After test, the DSC endothermic peak of the colloid was at 192.65°C, the width of the endothermic peak was 57.13, the exothermic peak was at 256.72°C, and the turbidity at 25°C to 95°C was 1.32 cm$^{-1}$.

Example 55

Casein was weighed and dissolved in 100 mL of deionized water, pH was adjusted to 7, and stirring was performed at 800 rpm for 3 h to prepare a 3% protein emulsion, which was sterilized with ultraviolet light for 15 min; the bacterial powder was stirred and dispersed in oil and fat (cocoa butter source, having DSC endothermic peak at around 30°C and DSC exothermic peak at around 200°C, with a melting curve slope of -2.45 and a viscosity of 28.0 mPa·s) at a mass ratio of 1:2, and stirred for 2-4 min, and the oil and fat dispersed with bacterial powder and the protein emulsion were emulsified at a mass ratio of 1:2 at 12000 rpm for 4 min to obtain a core material emulsion; sodium alginate powder and curdlan gum (1:10, w/w) were mixed and evenly dispersed in deionized water, and stirred at 800 rpm and 45°C for 30 min to obtain an outer layer colloid solution with a colloid mass concentration of 0.5%; the core material emulsion and the outer layer colloid solution were magnetically stirred at a mass ratio of 1:6 at 40°C and 1500 rpm for 20-30 s to obtain a mixed solution, which was then dropped into a 10% calcium chloride solution (pH 7) and reacted for 10 min to form microcapsules; the prepared microcapsules were placed in a 95°C water bath and heated for 1 min to cure into irreversible microcapsules, which were stored at 4°C.

The outer layer colloid solution in Example 55 was dropped into a 10% calcium chloride solution (pH 7) and reacted for 10 min to form microcapsules; the prepared microcapsules were placed in a 95°C water bath and heated for 1 min for curing, and the cured colloid was tested. After test, The results were basically consistent with those of Example 49.

Example 56

Whey protein was weighed and dissolved in 100 mL deionized water, pH was adjusted to 7, and stirring was performed at 800 rpm for 3 h to prepare a 3% protein emulsion, which was sterilizes with ultraviolet light for 15 min; the bacterial powder was stirred and dispersed in oil and fat (cocoa butter source, having DSC endothermic peak at around 30°C and DSC exothermic peak at around 200°C, with a melting curve slope of -2.45 and a viscosity of 28.0 mPa·s) at a mass ratio of 1:2, and stirred for 2-4 min, and the oil and fat dispersed with bacterial powder and the protein emulsion were emulsified at a mass ratio of 1:2 at 12000 rpm for 4 min to obtain a core material emulsion; sodium alginate powder and curdlan gum (1:10, w/w) were mixed evenly and dispersed in deionized water, and stirred at 800 rpm and 45°C for 30 min to obtain an outer layer colloid solution with a colloid mass concentration of 0.5%; the core material emulsion and the outer layer colloid solution were magnetically stirred at 40°C and 1500rpm for 20 s in a mass ratio of 1:6 to obtain a mixed solution, which was dropped into a 10% calcium chloride solution (pH 7) and reacted for 10 min to form microcapsules; the prepared microcapsules were placed in a 95°C water bath and heated for 1 min to cure into irreversible microcapsules, which were stored at 4°C.

The outer layer colloid solution in Example 56 was dropped into a 10% calcium chloride solution (pH 7) and reacted for 10 min to form microcapsules; the prepared microcapsules were placed in a 95°C water bath and heated for 1 min for curing, and the cured colloid was tested. After test, the results were basically consistent with those of Example 49.

Example 57

Soy protein isolate was weighed and dissolved in 100 mL of deionized water, pH was adjusted to 7, and stirring was performed at 800 rpm for 3 h to prepare a 3% protein emulsion, which was sterilized with ultraviolet light for 15 min; the bacterial powder was stirred and dispersed in oil and fat (cocoa butter source, having DSC endothermic peak at around 30°C and DSC exothermic peak at around 200°C, with a melting curve slope of -2.45 and a viscosity of 28.0 mPa·s) at a mass ratio of 1:2, and stirred for 2-4 min. The oil and fat dispersed with bacterial powder and the protein emulsion were emulsified at a mass ratio of 1:2 at 12000 rpm for 4 min to obtain a core material emulsion; sodium alginate powder and curdlan gum (1:10, w/w) were mixed evenly and dispersed in deionized water, and stirred at 800 rpm and 45°C for 30 min to obtain an outer layer colloid solution with a colloid mass concentration of 0.5%; the core material emulsion and the outer layer colloid solution were magnetically stirred at 40°C and 1500rpm for 20 s in a mass ratio of 1:6 to obtain a mixed solution, which was dropped into a 10% calcium chloride solution (pH 7) and reacted for 10 min to form microcapsules; the prepared microcapsules were placed in a 95°C water bath and heated for 1 min to cure into irreversible microcapsules, which were stored at 4°C.

The outer layer colloid solution in Example 57 was dropped into a 10% calcium chloride solution (pH 7) and reacted for 10 min to form microcapsules; the prepared microcapsules were placed in a 95°C water bath and heated for 1 min for curing, and the cured colloid was tested. After test, the results were basically consistent with those of Example 49.

The stability of the irreversible microcapsules obtained in Examples 41 to 57 and Comparative Examples 16 to 22 was tested, and the results are shown in Table 22.

High temperature shearing performance test method:

50ml of sterile deionized water was put in a beaker and kept warm in a water bath at 85°C. After the deionized water in the beaker was completely heated to 85°C, 2.5g of sample was added into the beaker and stirred at 1800rpm for 10min using a stirrer. The sample was taken for plate spreading.

75°C, 2 min: the sample was put in 75°C sterile deionized water for 2 min, taken out and subjected to plate spreading.

95°C, 1 min: the sample was put in 95°C sterile deionized water for 1min, taken out and subjected to plate spreading.

Gastrointestinal tract digestion:

Simulated gastric juice-containing 8.5g/L NaCl, 3g/L pepsin, pH=1.8
Simulated intestinal juice-containing 8.5g/L NaCl, 3g/L bile salt, 10g/L pancreatic enzyme, 10g/L trypsin, pH=6.5
18 ml of simulated gastric juice and simulated intestinal juice were put in 50mL conical flasks respectively, and kept warm in a 37°C shaking incubator for 20min. 2.0g of sample was weighed, put in simulated gastric juice, incubated at 37°C shaking incubator for 2h, filtered out, washed twice with sterile deionized water, then put in simulated intestinal juice, incubated at 37°C shaking incubator for 4h, washed twice with sterile deionized water, and subjected to plate spreading.

Table 22 Results of irreversible microcapsule stability test

| Sample | 75°C, 2min Δ bacteria count | 95°C, 1min Δ bacteria count | High temperature shearing Δ bacteria count | Gastrointestinal tract digestion Δ bacteria count |
|---|---|---|---|---|
| Example 41 | 1.8 | 1.8 | 1 | 0.7 |
| Example 42 | 1.7 | 1.7 | 1 | 0.8 |
| Example 43 | 1.6 | 1.5 | 0.9 | 0.6 |
| Example 44 | 1.4 | 1.4 | 0.8 | 0.5 |
| Example 45 | 1.8 | 1.8 | 1 | 0.7 |
| Example 46 | 1.7 | 1.7 | 1.1 | 0.8 |
| Example 47 | 1.4 | 1.5 | 0.8 | 0.5 |
| Example 48 | 1.4 | 1.5 | 0.8 | 0.5 |
| Example 49 | 1.9 | 1.8 | 1 | 0.9 |
| Example 50 | 1.8 | 1.9 | 1.1 | 1 |
| Example 51 | 1.6 | 1.6 | 1 | 0.7 |
| Example 52 | 1.4 | 1.5 | 0.7 | 0.6 |
| Example 53 | 1.5 | 1.6 | 0.8 | 0.7 |
| Example 54 | 1.4 | 1.5 | 0.6 | 0.7 |
| Example 55 | 1.2 | 1.3 | 0.7 | 0.6 |
| Example 56 | 1.3 | 1.5 | 0.8 | 0.5 |
| Example 57 | 1.4 | 1.5 | 0.8 | 0.6 |
| Comparative Example 16 | 2.4 | 2.5 | 1.3 | 1.2 |
| Comparative Example 17 | 2.6 | 2.7 | 1.5 | 1.4 |
| Comparative Example 18 | 2.2 | 2 | 1.4 | 1.4 |
| Comparative Example 19 | 2.3 | 2.1 | 1.5 | 1.6 |
| Comparative Example 20 | 2.3 | 2.5 | 1.6 | 1.4 |
| Comparative Example 21 | 2.2 | 2.4 | 1.1 | 1.6 |
| Comparative Example 22 | 2.5 | 2.3 | 1.3 | 1.5 |

**Claims**

1.   A microcapsule, comprising a core material and a wall material wrapping the core material; wherein the wall material comprises a colloid; the colloid has a differential scanning calorimetry curve with an endothermic peak between 190°C to 210°C and an exothermic peak between 250°C to 281°C.

2. The microcapsule according to claim 1, wherein the endothermic peak has a width of 57 to 70°C; and/or,

the colloid has a turbidity of 1.3 to 1.5 cm$^{-1}$ at 25°C to 95°C; and/or,
the colloid comprises a thermal gel, preferably further comprises alginate and/or its salt; and/or,
the thermal gel is one or more selected from the group consisting of curdlan gum, konjac gum and gellan gum.

3. The microcapsule according to claim 1, wherein the colloid is obtained by heat treatment; and/or, the heat treatment is performed at a temperature of 75°C to 95°C; and/or, a calcification cross-linking step is conducted before the heat treatment.

4. The microcapsule according to claim 1, wherein a mass ratio of the core material to the colloid is 5.56:1 to 1.59:1 based on a mass ratio of dry matter.

5. The microcapsule according to claim 1, wherein a mass percentage of the colloid is 0.2% to 7% based on the mass of the microcapsule.

6. The microcapsule according to claim 1, wherein the core material comprises oil and fat composition; the oil and fat composition has a differential scanning calorimetry curve with an endothermic peak between 35°C to 47°C and an exothermic peak between 220°C to 250°C;
the oil and fat composition has a solid fat content curve with a slope of -3.0 to -1.0 at 20°C to 50°C.

7. The microcapsule according to claim 6, wherein the oil and fat composition has a viscosity value of 20 to 90 mPa·s at 95°C.

8. The microcapsule according to claim 6, wherein the oil and fat composition comprises one or more selected from the group consisting of a mixed oil, a mixed oil after transesterification, an intermediate fractionation oil and a hydrogenated oil;
the mixed oil comprises an unsaturated fatty acid oil and a saturated fatty acid oil; the unsaturated fatty acid oil has a content of unsaturated fatty acid of greater than 50%; and the saturated fatty acid oil has a content of saturated fatty acid of greater than 50%.

9. The microcapsule according to claim 8, wherein a mass ratio of the unsaturated fatty acid oil to the saturated fatty acid oil in the mixed oil is (10 to 70): (90 to 30).

10. The microcapsule according to claim 6, wherein a mass of the oil and fat composition is 5% to 17% of the mass of the microcapsule.

11. The microcapsule according to claim 1, wherein the core material comprises oil and fat; the oil and fat has a differential scanning calorimetry curve with an endothermic peak between 30°C and 48°C and an exothermic peak between 200°C and 245°C; and/or

the oil and fat has a solid fat content curve with a slope of -2.51 to -1.57 at 10°C to 40°C; and/or the oil and fat has a viscosity value of 23.900 mPa·s to 84.445 mPa·s at 95°C; and/or
the oil and fat is selected from a vegetable oil and fat and/or an animal oil and fat; and/or
the oil and fat is subjected to at least one process step selected from the group consisting of transesterification, fractionation and hydrogenation; and/or
a mass percentage of the oil is 5% to 17% based on the mass of the microcapsule.

12. The microcapsule according to claim 1, wherein the core material comprises at least one bioactive substance selected from the group consisting of probiotics, vitamins, polyunsaturated fatty acids, active proteins and natural pigments.

13. The microcapsule according to claim 1, wherein a protein coating layer is provided between the core material and the wall material.

14. The microcapsule according to claim 13, wherein a protein in the protein coating layer is one or more selected from the group consisting of casein, pea protein and whey protein; and/or
based on the mass of the microcapsule, a mass percentage of the mass of the protein coating layer to the mass of the microcapsule is 1% to 40%.

15. A heat-resistant and shear-resistant microcapsule, comprising a core material, a protein coating layer wrapping the core material, and a wall material wrapping the protein coating layer; the core material comprises oil and fat; and the wall material is formed by heat-curing a colloid.

16. The microcapsule according to claim 15, wherein the microcapsule comprises at least one of the following features:

(1) the oil and fat have a differential scanning calorimetry curve with an endothermic peak between 30°C and 48°C and an exothermic peak between 200°C and 245°C;
(2) the oil and fat have a solid fat content curve with a slope of -2.51 to -1.57 at 10°C to 40°C;
(3) the oil and fat have a viscosity of 23.900 mPa·s to 84.445 mPa·s at 95°C;
(4) the oil and fat are selected from a vegetable oil and fat and/or an animal oil and fat;
(5) the oil and fat are subjected to at least one process step selected from the group consisting of transesterification, fractionation and hydrogenation; and
(6) a mass percentage of the oil and fat is 5% to 17% based on the mass of the microcapsule.

17. The microcapsule according to claim 15, wherein a protein in the protein coating layer is one or more selected from the group consisting of casein, pea protein and whey protein; and/or, based on the mass of the microcapsule, a mass percentage of the mass of the protein coating layer to the mass of the microcapsule is 1% to 40%.

18. The microcapsule according to claim 15, wherein the microcapsule comprises at least one of the following features:

(i) the wall material is formed by calcification, cross-linking and heat curing of the colloid;
(ii) the wall material has a differential scanning calorimetry curve with an endothermic peak between 190°C to 210°C and an exothermic peak between 250°C to 281°C;
(iii) the wall material has a turbidity of 1.3 to 1.5 cm$^{-1}$ at 25°C to 95°C;
(iv) the colloid comprises alginate and/or its salt and a gelling agent; and
(v) based on the mass of the microcapsule, a mass percentage of the colloid is 0.2% to 7%.

19. The microcapsule according to claim 18, wherein a mass ratio of the gelling agent to alginate and/or its salt is (1-20):1; preferably, the gelling agent is one or more selected from the group consisting of thermoreversible gel, curdlan gum, konjac gum and gellan gum.

20. The microcapsule according to claim 15, wherein the core material of the microcapsule comprises at least one bioactive substance selected from the group consisting of probiotics, vitamins, polyunsaturated fatty acids, active proteins and natural pigments.

21. A method for producing a heat-resistant and shear-resistant microcapsule, comprising steps of:

S1) mixing a bioactive substance with an oil and fat to obtain a core material;
S2) mixing and emulsifying the core material with a protein emulsion to obtain a core material emulsion;
S3) mixing and stirring the core material emulsion with a wall material solution to obtain a mixed solution; wherein the wall material solution comprises a colloid; and
S4) subjecting the mixed solution to heat treatment to obtain the microcapsule.

22. The production method according to claim 21, wherein a mass concentration of the protein emulsion is 1% to 5%; a mass ratio of the core material to the protein emulsion is 1:(1 to 10); and/or,
a mass concentration of the colloid in the wall material solution is 0.1% to 1%; a mass ratio of the core material emulsion to the wall material solution is 1:(2 to 7); and/or, the heat treatment is performed at a temperature of 75°C to 95°C; and/or, the heat treatment has a duration of 10 to 30 min.

23. Use of the heat-resistant and shear-resistant microcapsule according to any one of claims 15 to 20 or the heat-resistant and shear-resistant microcapsule produced by the production method according to any one of claims 21 to 22 as a food additive.

24. A food, comprising the heat-resistant and shear-resistant microcapsule according to any one of claims 15 to 20 or the heat-resistant and shear-resistant microcapsule produced by the production method according to any one of claims 21 to 23.

25. A high-temperature-resistant and shear-resistant colloid, wherein the colloid has a differential scanning calorimetry curve with an endothermic peak between 190°C and 210°C and an exothermic peak between 250°C and 281°C.

26. The colloid according to claim 25, wherein the endothermic peak has a width of 57 to 70°C; and/or,

the colloid has a turbidity of 1.3 to 1.5 cm$^{-1}$ at 25°C to 95°C; and/or,
the colloid comprises a thermal gel, preferably further comprising alginic acid and/or its salt; and/or, the thermal gel is one or more selected from the group consisting of curdlan gum, konjac gum and gellan gum.

27. The colloid according to claim 25, wherein the colloid is obtained by heat treatment; and/or, the heat treatment is performed at a temperature of 75°C to 95°C; and/or, a calcification cross-linking step is conducted before the heat treatment.

28. Use of the colloid according to any one of claims 25 to 27 in improving the heat resistance of a bioactive substance; preferably, the heat resistance is heat resistance of the bioactive substance in a shear environment; and the bioactive substance is a heat-sensitive substance.

29. A food, comprising the colloid according to any one of claims 25 to 27.

30. A microcapsule, comprising a core material and a wall material wrapping the core material; the wall material comprises the colloid according to any one of claims 25 to 27; preferably, a mass ratio of the core material to the colloid is 5.56:1 to 1.59:1 based on a mass ratio of dry matter.

31. A method for producing a microcapsule, comprising steps of:

S1) mixing a colloidal raw material with water to obtain a colloidal raw material solution;
S2) mixing and stirring a core material with the colloidal raw material solution to obtain a mixed solution; and
S3) subjecting the mixed solution to heat treatment to obtain the microcapsule;
preferably, a mass concentration of the colloidal raw material in the colloidal raw material solution is 0.1% to 1%;
preferably, a mass ratio of the core material to the colloidal raw material solution is 1:(2 to 7); preferably, the heat curing is performed at a temperature of 75°C to 95°C.

32. Use of the microcapsule according to claim 30 or the microcapsule produced by the production method according to claim 31 as a food additive.

33. Use of the microcapsule according to claim 30 or the microcapsule produced by the production method according to claim 31 in improving heat resistance of a bioactive substance; preferably, the heat resistance is heat resistance of the bioactive substance in a shear environment; and the bioactive substance is a heat-sensitive substance.

34. A food, comprising the microcapsule according to claim 30 or the microcapsule produced by the production method according to claim 31.

35. A heat-resistant and acid-resistant oil and fat composition, wherein the oil and fat composition has a differential scanning calorimetry curve withan endothermic peak between 35°C and 47°C and an exothermic peak between 220°C and 250°C; and
the oil and fat composition has a solid fat content curve of with a slope of -3.0 to -1.0 at 20°C to 50°C.

36. The oil and fat composition according to claim 35, wherein the oil and fat composition has a viscosity value of 20 to 90 mPa·s at 95°C.

37. The oil and fat composition according to claim 35, wherein the oil and fat composition comprises one or more selected from the group consisting of a mixed oil, a mixed oil after transesterification, an intermediate fractionation oil and a hydrogenated oil;
the mixed oil comprises an unsaturated fatty acid oil and a saturated fatty acid oil; the unsaturated fatty acid oil has a content of the unsaturated fatty acid of greater than 50%; the saturated fatty acid oil has a content of the saturated fatty acid of greater than 50%; preferably, a mass ratio of the unsaturated fatty acid oil to the saturated fatty acid oil in the mixed oil is (10-70): (90-30).

38. Use of the oil and fat composition according to any one of claims 35-37 in encapsulating a bioactive substance.

39. A food, comprising the oil and fat composition according to any one of claims 35-37.

40. A microcapsule, comprising a core material and a wall material wrapping the core material; the core material comprises the oil and fat composition according to any one of claims 35-37.

41. The microcapsule according to claim 40, wherein a mass of the oil and fat composition is 5% to 17% of the mass of the microcapsule;

preferably, a protein coating layer is provided between the core material and the wall material;
preferably, the wall material is formed by heat-curing the colloid; a differential scanning calorimetry curve of the wall material has an endothermic peak between 190°C and 210°C and an exothermic peak between 250°C and 281°C; a turbidity of the wall material at 25°C to 95°C is 1.3 to 1.5 cm$^{-1}$; the colloid comprises a gelling agent and alginic acid and/or its salt; a mass ratio of the gelling agent to alginic acid and/or its salt is (1 to 20): 1; the gelling agent is one or more selected from the group consisting of a thermoreversible gel, curdlan gum, konjac gum and gellan gum.

42. A method for producing a microcapsule, comprising:

S1) dispersing a bioactive substance in the oil and fat composition according to any one of claims 35 to 37 to obtain a core material;
S2) mixing and stirring the core material and a wall material solution to obtain a mixed solution; and
S3) heat curing the mixed solution to obtain the microcapsule.

43. Use of the microcapsule according to any one of claims 40 to 41 or the microcapsule produced by the production method according to claim 42 as a food additive.

44. A food, comprising the microcapsule according to any one of claims 40 to 41 or the microcapsule produced by the production method according to claim 42.

## INTERNATIONAL SEARCH REPORT

| International application No. |
|---|
| **PCT/CN2023/142218** |

| A. | CLASSIFICATION OF SUBJECT MATTER |
|---|---|

A23P 10/30(2016.01)i; A23P 10/35(2016.01)i; A23L 33/135(2016.01)i

According to International Patent Classification (IPC) or to both national classification and IPC

| B. | FIELDS SEARCHED |
|---|---|

Minimum documentation searched (classification system followed by classification symbols)

IPC:A23P,A23L

Documentation searched other than minimum documentation to the extent that such documents are included in the fields searched

Electronic data base consulted during the international search (name of data base and, where practicable, search terms used)

CNKI, CJFD, CNTXT, DWPI, ENTXTC, WPABSC: 胶, 胶体, 热凝胶, 热可逆凝胶, 可得然胶, 凝胶多糖, 魔芋胶, 结冷胶, 海藻酸钠, 钙, 蛋白, 油, 加热, 热处理, 微胶囊, 微囊, CaCl2, gum, colloid, thermogel, thermoreversible, curdlan gum, konjac gum, gellan gum, sodium alginate, calcium, protein, oil, microcapsule

| C. | DOCUMENTS CONSIDERED TO BE RELEVANT | | |
|---|---|---|---|
| Category* | Citation of document, with indication, where appropriate, of the relevant passages | | Relevant to claim No. |
| X | CN 112889938 A (HEZE ZONGHOO JIANYUAN BIOTECHNOLOGY CO., LTD.) 04 June 2021 (2021-06-04) claims 1-10 | | 1-17, 20-34 |
| Y | CN 112889938 A (HEZE ZONGHOO JIANYUAN BIOTECHNOLOGY CO., LTD.) 04 June 2021 (2021-06-04) claims 1-10 | | 18-19 |
| Y | CN 102578443 A (SHANGHAI TOPYUM BIO-TECHNOLOGY CO., LTD.) 18 July 2012 (2012-07-18) claims 1-7, and description, paragraphs 5-6 and 28-41 | | 18-19, 42 |
| Y | CN 111134334 A (INNER MONGOLIA MENGNIU DAIRY (GROUP) CO., LTD.) 12 May 2020 (2020-05-12) claims 1-12 | | 18-19 |
| X | CN 114431471 A (SINOFN (TIANJIN) PHARM-TECH CO., LTD. et al.) 06 May 2022 (2022-05-06) claims 1-13, and description, paragraph 29 | | 35-41, 43-44 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 March 2024** | **31 March 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/142218**

### C.    DOCUMENTS CONSIDERED TO BE RELEVANT

| Category* | Citation of document, with indication, where appropriate, of the relevant passages | Relevant to claim No. |
|---|---|---|
| Y | CN 114431471 A (SINOFN (TIANJIN) PHARM-TECH CO., LTD. et al.) 06 May 2022 (2022-05-06)<br>claims 1-13, and description, paragraph 29 | 42 |
| A | CN 108065443 A (XIAMEN KINGDOMWAY VITAMIN LTD. et al.) 25 May 2018 (2018-05-25)<br>claims 1-10 | 1-44 |

## INTERNATIONAL SEARCH REPORT
### Information on patent family members

International application No.

**PCT/CN2023/142218**

| Patent document cited in search report | | | Publication date (day/month/year) | Patent family member(s) | | | Publication date (day/month/year) |
|---|---|---|---|---|---|---|---|
| CN | 112889938 | A | 04 June 2021 | None | | | |
| CN | 102578443 | A | 18 July 2012 | None | | | |
| CN | 111134334 | A | 12 May 2020 | AU | 2020373489 | A1 | 19 May 2022 |
| | | | | WO | 2021082382 | A1 | 06 May 2021 |
| | | | | US | 2022395466 | A1 | 15 December 2022 |
| | | | | CA | 3156603 | A1 | 06 May 2021 |
| | | | | EP | 4042881 | A1 | 17 August 2022 |
| CN | 114431471 | A | 06 May 2022 | None | | | |
| CN | 108065443 | A | 25 May 2018 | CN | 108065443 | B | 28 February 2020 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211741593
- CN 202211734574
- CN 202211734622
- CN 201811161108
- GB 102472008 T
- GB 317432015 T

**Non-patent literature cited in the description**

- **BEICHEN WEST ROAD** ; **CHAOYANG DISTRICT**. Beijing, Institute of Microbiology. *Chinese Academy of Sciences*, 26 April 2018